# EUROPEAN PATENT APPLICATION

(11) **EP 4 434 988 A1**
(43) Date of publication of application: **25.09.2024**
(21) Application number: 22894906.1
(22) Date of filing: 17.11.2022
(51) Int. Cl.: C07D 471/10

(54) **ß-LACTAMASE INHIBITOR INTERMEDIATE AND PREPARATION METHOD THEREFOR**

(30) Priority: 17.11.2021 CN 202111364821; 17.11.2021 CN 202111364826
(71) Applicant: Evopoint Biosciences Co., Ltd., Suzhou, Jiangsu 215000 (CN)
(72) Inventor: WANG, Xufan, Suzhou, Jiangsu 215000 (CN); ZHANG, Huixin, Suzhou, Jiangsu 215000 (CN); WANG, Yanfei, Suzhou, Jiangsu 215000 (CN); WANG, Wengui, Suzhou, Jiangsu 215000 (CN); HU, Yuanyuan, Suzhou, Jiangsu 215000 (CN); DU, Guowen, Suzhou, Jiangsu 215000 (CN); HU, Hanwei, Suzhou, Jiangsu 215000 (CN)
(74) Representative: Michalski Hüttermann & Partner Patentanwälte mbB
(86) International application number: PCT/CN2022/132591
(87) International publication number: WO 2023/088375

(57) **Abstract**

Provided is a method for preparing an oxadiazazole compound and/or a tautomer thereof, comprising: (a), in a solvent, in the presence of a dehydrating agent and a base, subjecting a compound as represented by formula 18 and/or a tautomer thereof to a ring-closing reaction to obtain a compound as represented by formula 19 and/or a tautomer thereof; and/or (b), in a solvent, under acidic conditions, subjecting the compound as represented by formula 19 to a tautomerization reaction as shown to obtain a tautomer thereof, such as a compound as represented by formula 20. The method for preparing the β-lactamase inhibitor and the intermediate has a low cost, improves the repeatability and the scalability of key reactions, significantly increases the total yield and reduces the number of reaction steps as compared with the prior art, has the technical advantages of being green, safe, efficient and simple and convenient, and is suitable for industrial production.

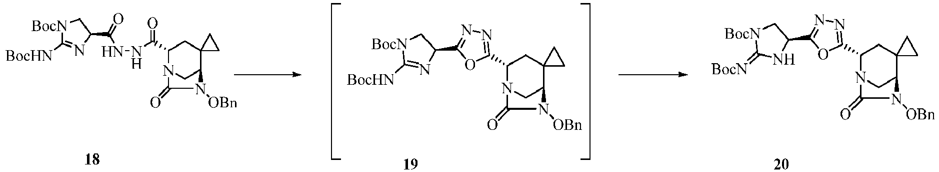

## Description

The present application claims priority to the Chinese Patent Application No. 202111364821X filed on November 17, 2021 and the Chinese Patent Application No. 2021113648262 filed on November 17, 2021, which are incorporated herein by reference in their entirety.

### TECHNICAL FIELD

The present invention relates to a β-lactamase inhibitor intermediate and a preparation method therefor.

### BACKGROUND

β-lactam antibiotics are the first antibiotics introduced for clinical use. The development of β-lactam antibiotics has been accelerated since the successful application of penicillin G as the first β-lactam antibiotic in clinic practice. β-lactam antibiotics with different structures have been developed and widely applied in clinic practice with excellent results. However, bacterial cells can produce β-lactamases which inactivate the antibiotics, resulting in bacterial resistance to β-lactam antibiotics. β-lactamases are enzymes that catalyze the hydrolysis of β-lactam rings, which inactivates the antibacterial activity of β-lactam antibiotics and allows bacteria to develop resistance to β-lactam antibiotics. β-lactamases can be divided into class A, B, C, D, etc. according to the amino acid sequence differences in the molecular structure. Class A β-lactamases preferably hydrolyze penicillin antibiotics. Class B β-lactamases can hydrolyze various β-lactam antibiotics, including carbapenem antibiotics. Class C β-lactamases can more effectively hydrolyze cephalosporin antibiotics. Class D β-lactamases are more inclined to hydrolyze oxacillin and o-cloxacillin. The resistance of bacteria, particularly gram-negative bacteria, to β-lactam antibiotics is typically mediated by β-lactamases.

Inhibition of β-lactamases can delay or inhibit the degradation of β-lactam antibiotics and restore the susceptibility of β-lactam antibiotic-resistant bacteria to β-lactam antibiotics. At present, in clinical practice, the hydrolytic activity of β-lactamases to β-lactam antibiotics can be inactivated by combining β-lactamases with β-lactam antibiotics, so that the susceptibility of bacteria to β-lactam antibiotics is enhanced, and the problem of drug resistance is reduced or overcome. The prior art discloses various bacterial β-lactamase inhibitors, for example, diazaspiro[bicyclo[3.2.1]octane compounds disclosed in WO2013149121A1, WO2014141132A1, US20130296290A1, WO2013030735A1, WO2015110963A1, WO2015150890A1, WO2015159265A1, WO2015173663, WO2015173665A1, and WO2017055922A1. Furthermore, various β-lactamase inhibitors are commercially available, such as Clavulanic Acid, Tazobactam, Avibactam, and Relebactam. However, the inhibitory effects of the above β-lactamase inhibitors on β-lactamases are still not entirely satisfactory. Therefore, there is currently an urgent need for novel β-lactamase inhibitors to be able to treat infections caused by β-lactam antibiotic-resistant bacteria in combination with β-lactam antibiotics.

The Patent Application No. WO2019144912A1 discloses a β-lactamase inhibitor of formula I below, which has a good antibacterial activity:

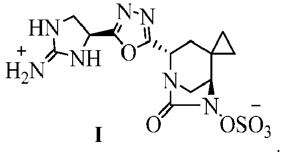

This patent application discloses a reaction route, wherein
(1) in the first step, a condensing agent such as HATU is used for condensation, but the HATU condensing agent has a very high cost and is only suitable for small samples; (2) SM-6 and SM-7 are unstable in acidic systems; and (3) a total of 9 steps of reactions are required starting from the starting material SM-1.

Therefore, the original preparation method has the disadvantages of a long reaction route and harsh reaction conditions, thus being not suitable for large-scale industrial production, and the yield needs to be further improved.

### SUMMARY

The present invention aims to solve the technical problems to overcome the technical defects of the existing preparation methods for the β-lactamase inhibitor of formula I, such as lengthy synthetic route, low overall yield, use of toxic, harmful, hazardous or expensive reagents, harsh reaction conditions, and/or poor reaction repeatability and scalability, etc., which are not suitable for large-scale synthesis. Therefore, the present invention provides a β-lactamase inhibitor intermediate, a preparation method, and an intermediate. Compared with the prior art, the preparation method provided by the present invention has the advantages that the starting materials are cheap and readily available, toxic, harmful and/or hazardous reagents and harsh reaction conditions are avoided, the number of reaction steps are significantly reduced, the overall yield is significantly increased, and the reaction repeatability and scalability are significantly improved, so that the preparation method is more suitable for industrial synthesis.

The present invention solves the above technical problems by the following technical solutions.

The present invention provides a preparation method for a diazabicyclooctane compound of formula 8, comprising the following step: adding a mixture of triphosgene and a solvent to a mixture of a compound of formula 7 and/or a salt thereof, a base and a solvent to conduct an amidation ring-closing reaction as shown below to give the diazabicyclooctane compound of formula 8:

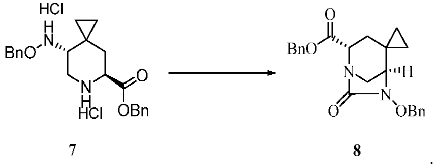

The solvent may be one or more of acetonitrile, a halogenated hydrocarbon solvent (e.g., dichloromethane), and an aromatic hydrocarbon solvent (e.g., toluene). The solvent may be used in an amount that does not affect the reaction, for example, the mass percentage of triphosgene in the mixture of the triphosgene and the solvent may be 10%. In the mixture of the compound of formula 7 and/or the salt thereof, the base and the solvent, the volume-to-mass ratio of the solvent to the compound of formula 7 may be 3 mL/g.

The salt of the compound of formula 7 may be a hydrochloride, for example, 2 equivalents of hydrochloric acid.

The base may be an organic base, such as *N*,*N*-diisopropylethylamine (DIPEA) and/or triethylamine.

The molar ratio of the base to the compound of formula 7 may be 4:1; or the molar ratio of the base to the salt of the compound of formula 7 may be 6: 1.

The molar ratio of the triphosgene to the compound of formula 7 and/or the salt thereof may be 0.35: 1 to 0.5:1, such as 0.38: 1 to 0.4:1.

The amidation ring-closing reaction may be conducted at a temperature of -10 °C to 0 °C.

The amidation ring-closing reaction is preferably conducted under an inert gas, such as nitrogen or argon.

The preparation method may comprise the following steps: adding the base to the mixture of the compound of formula 7 and/or the salt thereof and the solvent at an internal temperature of 10 °C to 30 °C, and then adding a solution of 10% triphosgene in the solvent to conduct the amidation ring-closing reaction as shown to give the diazabicyclooctane compound of formula 8.

The progress of the amidation ring-closing reaction may be detected by a conventional monitoring method in the art (e.g., TLC, HPLC, or NMR). The end point of the reaction is generally considered the point at which the compound of formula 7 disappears or no longer reacts. The amidation ring-closing reaction is preferably conducted for a period of 8 h to 15 h, such as 9-10 h.

The step may also comprise a post-treatment, wherein the post-treatment may comprise the following steps: after the amidation reaction is completed, adding water and methyl *tert*-butyl ether, mixing, then washing and separating the obtained organic phase, concentrating the organic phase, adding acetone, and concentrating. The quenching may be conducted by adding water and methyl tert-butyl ether in amounts of 5 times and 7 times the mass of the compound of formula 7, respectively, with the temperature controlled at 15-25 °C; the washing may be conducted by sequentially adding a mixed aqueous solution of 5% sodium carbonate and 5% sodium chloride and a saturated aqueous sodium chloride solution in amounts of 8 times and 5 times the mass of the compound of formula 7, respectively. The concentration may be conducted under reduced pressure with the temperature controlled to be no higher than 55 °C.

In a certain embodiment, the preparation method for the diazabicyclooctane compound of formula 8 also comprises a preparation method for the compound of formula 7 and/or a hydrochloride thereof,

which comprises the following steps:
(1) in a solvent, in the presence of sulfuric acid, reacting a compound of formula 6 with an acid to give a mixture A; and
(2) adding a reducing agent to the mixture A to conduct a reduction reaction to give the compound of formula 7:

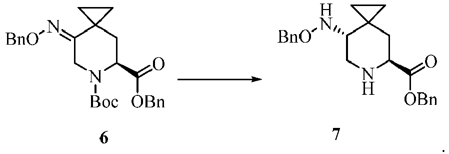

The solvent may be an ester solvent, such as ethyl acetate. The solvent may be used in an amount that does not affect the reaction, for example, the volume-to-mass ratio of the solvent to the compound of formula 6 may be 9 mL/g to 15 mL/g, such as 12 mL/g.

The molar ratio of the sulfuric acid to compound 6 may be 4:1 to 12:1, such as 12:1.

The sulfuric acid may be added in the form of a solution of concentrated sulfuric acid in the solvent, wherein in the solution, the mass ratio of the concentrated sulfuric acid to the solvent may be 1.4:1.

The reaction in step (1) may be conducted at a temperature of -45 °C to -5 °C, such as -35 °C to -25 °C.

The progress of the reaction in step (1) may be detected by a conventional monitoring method in the art (e.g., TLC, HPLC, or NMR), and the end point of the reaction is generally considered the point at which the compound of formula 6 disappears or no longer reacts. The reaction is preferably conducted for a period of 10 h to 30 h, such as 21 h.

After the reaction in step (1) is completed, the reduction reaction in step (2) may be directly conducted without a post-treatment to prepare compound 7.

The reducing agent may be sodium borohydride, borane-methyl sulfide complex, and lithium triethylborohydride, for example, sodium borohydride.

The molar ratio of the reducing agent to the compound of formula 6 may be 4:1 to 6:1, such as 4:1.

The reduction reaction may be conducted at a temperature of -70 °C to -35 °C, such as -70 °C to -60 °C.

The progress of the reduction reaction may be detected by a conventional monitoring method in the art (e.g., TLC, HPLC, or NMR), and the end point of the reaction is generally considered the point at which the compound of formula 7 no longer increases. The reduction reaction is preferably conducted for a period of 2 h to 10 h, such as 4 h.

Step (2) may also comprise a post-treatment, wherein the post-treatment may comprise the following steps: after the reduction reaction is completed, adding water to quench the reaction, adding an ammonium hydroxide solution to neutralize the reaction, separating the obtained organic phase, concentrating, adding ethanol, and concentrating to give compound 7;

or further adding ethanol and hydrogen chloride to form a salt, crystallizing, then filtering, washing a filter cake with ethanol, and drying (at 50 °C under vacuum) to give the hydrochloride of compound 7. The quenching may be conducted at 0 °C to 10 °C; the concentration of the ammonium hydroxide solution may be 25%; the hydrogen chloride may be a solution of hydrogen chloride in ethanol, such as a 4 M solution of hydrogen chloride in ethanol.

The preparation method may also comprise a method for preparing compound 6, which comprises the following step: in a solvent, in the presence of a base, subjecting a compound of formula 5 to a Schiff base reaction with O-phenylhydroxylamine or a salt thereof to give compound 6:

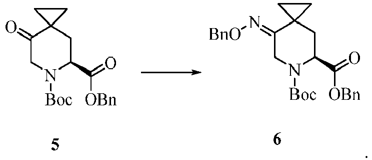

The solvent may be an alcohol solvent and/or an ester solvent, wherein the alcohol solvent may be one or more of ethanol, isopropanol, *tert*-butanol, and *tert*-amyl alcohol, and the ester solvent may be ethyl acetate. The solvent may be used in an amount that does not affect the reaction, for example, the volume-to-mass ratio of the solvent to the compound of formula 5 may be 8 mL/g to 12 mL/g.

The molar ratio of the compound of formula 5 to the *O*-phenylhydroxylamine or the salt thereof may be 1.15:1 to 1.5:1, such as 1.5:1.

The salt of the *O*-phenylhydroxylamine may be a hydrochloride of the *O-*phenylhydroxylamine.

The base may be an alkali metal bicarbonate, such as sodium bicarbonate.

The molar ratio of the base to the salt of the *O*-phenylhydroxylamine may be 2:1.5.

The molar ratio of the base to the compound of formula 5 may be 1.5:1 to 2:1, such as 2:1.

The Schiff base reaction is preferably conducted under an inert gas, such as nitrogen or argon.

The Schiff base reaction may be conducted at a temperature of 15 °C to 45 °C, such as 15 °C to 25 °C.

Preferably, the method for preparing compound 6 comprises the following steps: in a solvent, at 15 °C to 25 °C, adding the base to a mixture of the *O*-phenylhydroxylamine or the salt thereof and a solvent, and then adding a mixture of the compound of formula 5 and a solvent to conduct the Schiff base reaction to give compound 6.

The progress of the Schiff base reaction may be detected by a conventional monitoring method in the art (e.g., TLC, HPLC, or NMR), and the end point of the reaction is generally considered the point at which the compound of formula 5 disappears or no longer reacts. The ring-closing reaction is preferably conducted for a period of 1 h to 10 h, such as 3 h.

The step may also comprise a post-treatment, wherein the post-treatment may comprise the following steps: after the Schiff base reaction is completed, concentrating, adding methyl *tert*-butyl ether and water for extraction, adding an aqueous citric acid solution to an organic phase for washing, then adding an aqueous sodium bicarbonate solution to the organic phase for washing, concentrating the organic phase, adding ethyl acetate, and concentrating under reduced pressure to give compound 6. The aqueous citric acid solution may be a 10% aqueous citric acid solution, and the washing may be conducted twice; the aqueous sodium bicarbonate solution may be a 5% aqueous sodium bicarbonate solution; the concentration may be conducted under reduced pressure with the temperature controlled below 50 °C.

The preparation method may further comprise a method for preparing compound 5, which comprises the following step:
in a solvent, in the presence of a catalyst and a ligand, subjecting a compound of formula 4 to a ring-closing reaction as shown below to give compound 5:

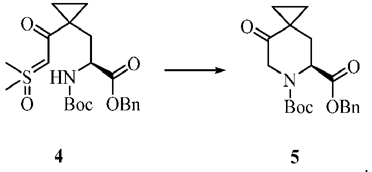

The solvent may be one or more of an aromatic hydrocarbon solvent, an alcohol solvent, an ether solvent, an amide solvent, and an ester solvent, wherein the aromatic hydrocarbon solvent may be toluene, the alcohol solvent may be *tert*-amyl alcohol and/or *tert-*pentanol, the ether solvent may be cyclopentyl methyl ether and/or tetrahydrofuran, the amide solvent may be DMAc and/or DMF, and the ester solvent may be isopropyl acetate; for example, the solvent may be toluene. The solvent may be used in an amount that does not affect the reaction, for example, the volume-to-mass ratio of the solvent to the compound of formula 4 may be 2 mL/g to 3 mL/g.

The catalyst may be one or more of chloro(cyclopentadienyl)bis(triphenylphosphine)ruthenium(II), chloro(1,5-cyclooctadiene)iridium(I) dimer, (1,5-cyclooctadiene)(methoxy)iridium(I) dimer, copper acetate, copper(I) iodide, palladium(II) acetate, *trans*-dichlorobis(acetonitrile)palladium(II), and palladium(II) trifluoroacetate, for example, chloro(cyclopentadienyl)bis(triphenylphosphine)ruthenium(II).

The molar ratio of the catalyst to the compound of formula 4 may be 0.01 to 0.1, such as 0.015:1.

Preferably, in the ring-closing reaction, a ligand conventional in the art for such reactions may also be added, wherein the ligand may be a phosphine ligand, such as triphenylphosphine. The molar ratio of the ligand to the compound of formula 4 may be 0.01 to 0.1, such as 0.04: 1.

The ring-closing reaction is preferably conducted under an inert gas, such as nitrogen or argon.

The ring-closing reaction may be conducted at a temperature of 60 °C to 110 °C, such as 95 °C to 105 °C.

The progress of the ring-closing reaction may be detected by a conventional monitoring method in the art (e.g., TLC, HPLC, or NMR), and the end point of the reaction is generally considered the point at which the compound of formula 4 disappears or no longer reacts. The ring-closing reaction is preferably conducted for a period of 1 h to 5 h, such as 2 h.

The step may also comprise a post-treatment, wherein the post-treatment may comprise the following steps: after the ring-closing reaction is completed, adding water for washing, and concentrating an organic phase.

The preparation method may also comprise a method for preparing compound 4, which comprises the following step:
in a solvent, in the presence of a base, subjecting a compound of formula 3 to a ring-opening reaction as shown below with trimethylsulfoxonium iodide to give compound 4:

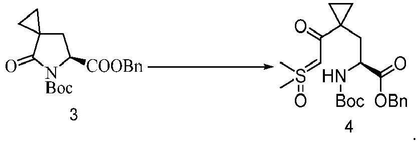

The solvent may be a mixture of an ether solvent and dimethyl sulfoxide, wherein the ether solvent may be tetrahydrofuran, and the volume ratio of the ether solvent to the dimethyl sulfoxide may be 1:1.2 to 3:1, such as 5:3.

The base may be an alkali metal alkoxide, such as potassium *tert*-butoxide.

The molar ratio of the base to the compound of formula 3 may be a molar ratio conventional in the art for such reactions, for example, 1:1 to 1.4:1, such as 1.15:1 to 1.2:1.

The molar ratio of the trimethylsulfoxonium iodide to the compound of formula 3 may be a molar ratio conventional in the art for such reactions, for example, 1:1 to 1.4:1, such as 1.15:1 to 1.2:1.

The ring-opening reaction is preferably conducted under an inert gas, wherein the inert gas may be argon or nitrogen.

The ring-opening reaction may be conducted at a temperature of -20 to 30 °C.

The step preferably comprises: mixing the base and the trimethylsulfoxonium iodide in a portion of an ether solvent and dimethyl sulfoxide, and then sequentially adding the remaining portion of the ether solvent and the compound of formula 3 to conduct the ring-opening reaction. The mixing may be conducted at a temperature of 20-30 °C; the ring-opening reaction may be conducted at a temperature of -10 to 0 °C.

The progress of the ring-opening reaction may be detected by a conventional monitoring method in the art (e.g., TLC, HPLC, or NMR), and the end point of the reaction is generally considered the point at which the compound of formula 3 disappears or no longer reacts. The ring-opening reaction is preferably conducted for a period of 5 h to 24 h, such as 12 h.

The step may also comprise a post-treatment, wherein the post-treatment may comprise the following steps: after the ring-opening reaction is completed, sequentially adding an organic solvent and a 5% ammonium chloride solution, performing layer separation, washing an aqueous phase with an organic solvent, washing a combined organic phase with a 5% to 2% sodium chloride solution, drying, filtering, and concentrating the organic phase.

The preparation method may also comprise a method for preparing the compound of formula 3, which comprises the following step:
in a solvent, in the presence of a Zn/Cu or Zn/Ag reagent, subjecting a compound of formula 59 to a cyclopropanation reaction as shown below with a methylating reagent to give the compound of formula 3:

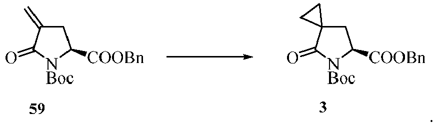

The solvent may be an amide solvent, such as DMF. The solvent may be used in an amount that does not affect the reaction, for example, the volume-to-mass ratio of the solvent to the compound of formula 59 may be 5 mL/g to 30 mL/g, such as 10 mL/g.

The molar ratio of the Zn/Cu or Zn/Ag reagent to the compound of formula 59 may be 0.5:1 to 3:1, such as 2:1.

The methylating reagent may be dibromomethane and/or diiodomethane.

The molar ratio of the methylating reagent to the compound of formula 59 may be 1.5:1 to 3:1, such as 2:1.

The cyclopropanation reaction is preferably conducted under an inert gas, such as nitrogen or argon.

The cyclopropanation reaction may be conducted at a temperature of 15 °C to 45 °C, such as 30 °C to 40 °C.

The progress of the cyclopropanation reaction may be detected by a conventional monitoring method in the art (e.g., TLC, HPLC, or NMR), and the end point of the reaction is generally considered the point at which the compound of formula 59 disappears or no longer reacts. The cyclopropanation reaction is preferably conducted for a period of 10 h to 30 h, such as 17 h.

The step may also comprise a post-treatment, wherein the post-treatment may comprise the following steps: after the cyclopropanation reaction is completed, sequentially adding ethyl acetate and an aqueous ammonium chloride solution (e.g., 5%), filtering (through celite), separating, washing an aqueous phase with ethyl acetate, washing a combined organic phase with an aqueous sodium chloride solution (e.g., 5%), concentrating, adding methanol and water to precipitate a solid, filtering, and drying.

In a certain embodiment, the preparation method also comprises a method for preparing the compound of formula 59, which comprises the following step:
in a solvent, in the presence of a base, an aqueous formaldehyde solution or paraformaldehyde, and a phase transfer catalyst, subjecting a compound of formula 58 to a reaction as shown below to give the compound of formula 59:

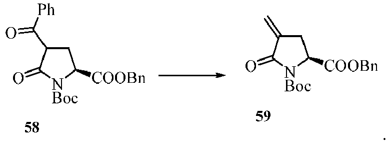

The solvent may be one or more of an ether solvent, a halogenated hydrocarbon solvent, a nitrile solvent, a ketone solvent, an ester solvent, and *N*,*N*-dimethylformamide, wherein the ether solvent may be tetrahydrofuran and/or 1,4-dioxane, the halogenated hydrocarbon solvent may be dichloromethane, the ester solvent may be ethyl acetate, the nitrile solvent may be acetonitrile, and the ketone solvent may be acetone; for example, the solvent may be dichloromethane. The solvent may be used in an amount that does not affect the reaction, for example, the volume-to-mass ratio of the solvent to the compound of formula 58 may be 8 mL/g to 12 mL/g.

The phase transfer catalyst may be a quaternary ammonium salt, such as tetrabutylammonium iodide (TBAI) and/or tetrabutylammonium fluoride trihydrate (TBAF·3H₂O). The molar ratio of the phase transfer catalyst to the compound of formula 58 may be 0.04:1 to 0.05:1, such as 0.045:1.

The base may be an inorganic base and/or an organic base, wherein the inorganic base may be one or more of an alkali metal carbonate, an alkali metal bicarbonate, and an alkali metal alkoxide, wherein the alkali metal carbonate may be potassium carbonate and/or cesium carbonate, the alkali metal bicarbonate may be potassium bicarbonate and/or sodium bicarbonate, and the alkali metal alkoxide may be potassium *tert*-butoxide; the organic base may be triethylamine and/or diisopropylamine; for example, the base may be potassium carbonate.

The molar ratio of the base to the compound of formula 58 may be 2:1 to 3:1.

The molar ratio of formaldehyde in the aqueous formaldehyde solution or the paraformaldehyde to the compound of formula 58 may be 1:1 to 6:1, such as 3.4:1.

Preferably, the base and the aqueous formaldehyde solution or the paraformaldehyde are added in portions, for example, in 3 equal portions.

The reaction is preferably conducted under an inert gas, such as nitrogen or argon.

The reaction may be conducted at a temperature of 15 °C to 55 °C, such as 35 °C to 45 °C.

The progress of the reaction may be detected by a conventional monitoring method in the art (e.g., TLC, HPLC, or NMR), and the end point of the reaction is generally considered the point at which the compound of formula 58 disappears or no longer reacts. The reaction is preferably conducted for a period of 10 h to 30 h, such as 15 h.

The step may also comprise a post-treatment, wherein the post-treatment may comprise the following steps: after the reaction is completed, adding a 5% aqueous ammonium chloride solution to quench the reaction, washing and separating the obtained organic phase, and concentrating.

The preparation method may also comprise a method for preparing the compound of formula 58, which comprises the following step:
in a solvent, in the presence of a base, subjecting a compound of formula 57 to a benzoylation reaction as shown below with benzoyl chloride to give the compound of formula 58:

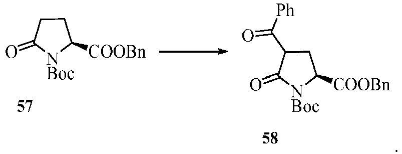

The solvent may be one or more of a ketone solvent, a nitrile solvent, an ether solvent, an ester solvent, and a halogenated hydrocarbon solvent, wherein the ketone solvent may be acetone, the nitrile solvent may be acetonitrile, the ether solvent may be tetrahydrofuran, the ester solvent may be ethyl acetate, and the halogenated hydrocarbon solvent may be dichloromethane; for example, the solvent may be tetrahydrofuran. The solvent may be used in an amount that does not affect the reaction, for example, the volume-to-mass ratio of the solvent to the compound of formula 57 may be 5 mL/g to 10 mL/g.

The molar ratio of the benzoyl chloride to the compound of formula 57 may be 1.05:1 to 1.1:1.

The base may be an alkali metal alkoxide and/or lithium bis(trimethylsilyl)amide (LiHMDS), wherein the alkali metal alkoxide may be one or more of sodium *tert*-butoxide, lithium *tert*-butoxide, and potassium *tert*-butoxide.

The molar ratio of the base to the compound of formula 57 may be 1.4:1 to 2.5:1.

The benzoylation reaction is preferably conducted under an inert gas, such as nitrogen or argon.

The benzoylation reaction may be conducted at a temperature of -65 °C to 25 °C, such as -15 °C to -5 °C.

The progress of the benzoylation reaction may be detected by a conventional monitoring method in the art (e.g., TLC, HPLC, or NMR), and the end point of the reaction is generally considered the point at which the compound of formula 57 disappears or no longer reacts. The benzoylation reaction is preferably conducted for a period of 1 h.

Preferably, the method for preparing the compound of formula 58 comprises the following step: at -15 °C to -5 °C, sequentially adding the base and the benzoyl chloride to a mixture of the compound of formula 57 and the solvent to conduct the benzoylation reaction to give the compound of formula 58.

The step may also comprise a post-treatment, wherein the post-treatment may comprise the following steps: after the benzoylation reaction is completed, adding a 10% aqueous citric acid solution to quench the reaction, washing and separating the obtained organic phase, and concentrating.

In a certain embodiment, the preparation method further comprises a preparation method for the compound of formula 57, which comprises the following step:
in a solvent, in the presence of a base and a phase transfer catalyst, subjecting a compound of formula 56 to a benzyl esterification reaction as shown below with benzyl bromide to give the compound of formula 57:

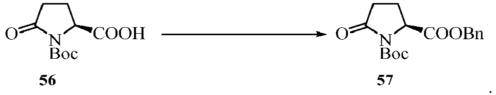

The operations and conditions for the benzyl esterification reaction may be those conventional in the art for such reactions; for example, the solvent may be an ester solvent, such as ethyl acetate. The base may be an alkali metal carbonate, such as potassium carbonate. The phase transfer catalyst may be tetrabutylammonium bromide (TBAB).

The present invention provides a preparation method for a diazabicyclooctane compound of formula 7, comprising the following steps:
(1) in a solvent, in the presence of sulfuric acid, reacting a compound of formula 6 with an acid to give a mixture A; and
(2) adding a reducing agent to the mixture A to conduct a reduction reaction to give the compound of formula 7:

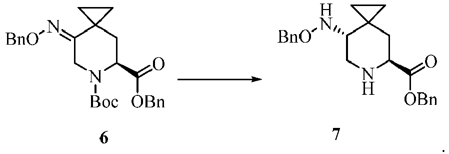

The operations and conditions in the preparation method may be the same as those in the preparation of the compound of formula 7 in the preparation method for the diazabicyclooctane compound of formula 8 described above.

The present invention provides a preparation method for a compound of formula 6, comprising the following step:
in a solvent, in the presence of a base, subjecting a compound of formula 5 to a Schiff base reaction with *O*-phenylhydroxylamine or a salt thereof to give the compound of formula 6:

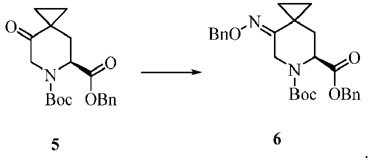

The operations and reaction conditions in the preparation method may be the same as those in the preparation of the compound of formula 6 in the preparation method for the diazabicyclooctane compound of formula 8 described above.

The present invention provides a preparation method for a compound of formula 5, comprising the following step:
in a solvent, in the presence of a catalyst and a ligand, subjecting a compound of formula 4 to a ring-closing reaction as shown below to give the compound of formula 5:

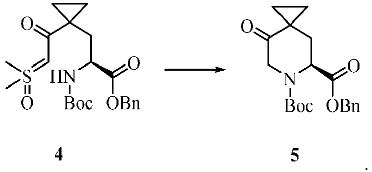

The operations and reaction conditions in the preparation method may be the same as those in the preparation of the compound of formula 5 in the preparation method for the diazabicyclooctane compound of formula 8 described above.

The present invention provides a preparation method for a compound of formula 3, comprising the following step:
in a solvent, in the presence of a Zn/Cu or Zn/Ag reagent, subjecting a compound of formula 59 to a cyclopropanation reaction as shown below with a methylating reagent to give the compound of formula 3:

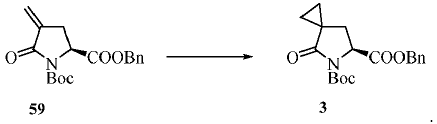

The operations and reaction conditions in the preparation method may be the same as those in the preparation of the compound of formula 3 in the preparation method for the diazabicyclooctane compound of formula 8 described above.

The present invention provides a preparation method for a compound of formula 59, comprising the following step:
in a solvent, in the presence of a base, an aqueous formaldehyde solution or paraformaldehyde, and a phase transfer catalyst, subjecting a compound of formula 58 to a reaction as shown below to give the compound of formula 59:

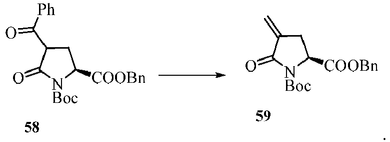

The operations and reaction conditions in the preparation method may be the same as those in the preparation of the compound of formula 59 in the preparation method for the diazabicyclooctane compound of formula 8 described above.

The present invention further provides a preparation method for a compound of formula 58, comprising the following step:
in a solvent, in the presence of a base, subjecting a compound of formula 57 to a benzoylation reaction as shown below with benzoyl chloride to give the compound of formula 58:

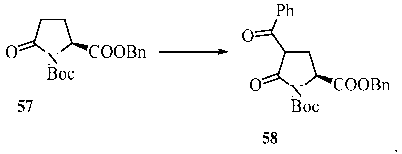

The operations and reaction conditions in the preparation method are the same as those in the preparation method for the compound of formula 58 in the preparation method for the diazabicyclooctane compound of formula 8 described above.

The present invention provides a compound of formula 5, 6 or 58:

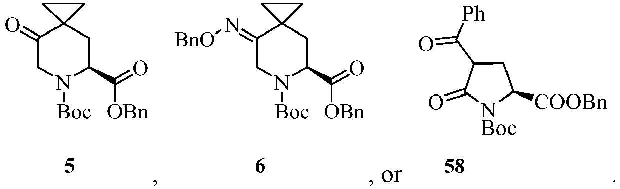

The present invention further provides a preparation method for an oxadiazazole compound and/or a tautomer thereof, comprising the following step (a) and/or step (b):
step (a):
   in a solvent, in the presence of a dehydrating agent and a base, subjecting a hydrazide compound of formula 18 and/or a tautomer thereof to a ring-closing reaction as shown to give an oxadiazazole compound of formula 19 and/or a tautomer thereof: 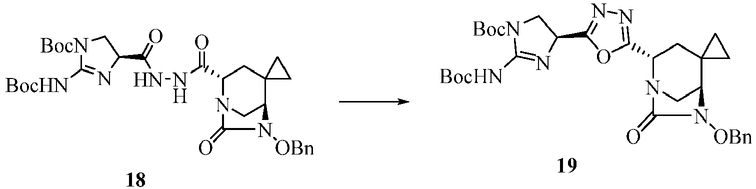
step (b): in an organic solvent and water, under an acidic condition, subjecting a hydrazide compound of formula 19 to a tautomerization reaction as shown to give a tautomer thereof, namely an oxadiazazole compound of formula 20:

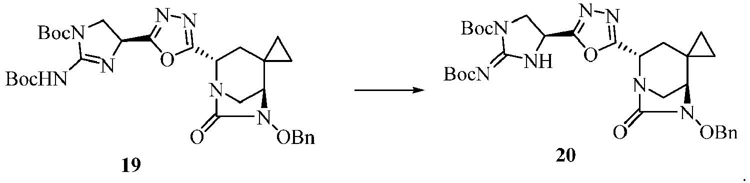

The tautomer of the hydrazide compound of formula 18 is as shown in formula 18', and the tautomer of the oxadiazazole compound of formula 19 is as shown in formula 20:

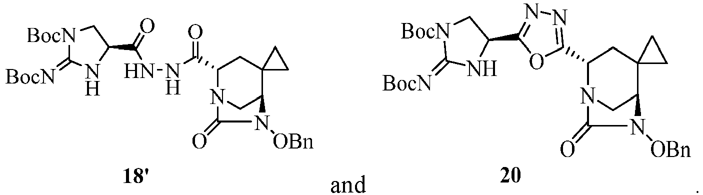

In step (a), the solvent may be one or more of an aromatic hydrocarbon solvent, an ether solvent, an ester solvent, and a halogenated hydrocarbon solvent, wherein the aromatic hydrocarbon solvent may be toluene, the ether solvent may be tetrahydrofuran, the ester solvent may be ethyl acetate, and the halogenated hydrocarbon solvent may be dichloromethane. The solvent may be used in an amount that does not affect the reaction, for example, the volume-to-mass ratio of the solvent to the compound of formula 18 may be 6.5 mL/g to 10 mL/g.

In step (a), the dehydrating agent may be Burgess reagent (methyl *N-*(triethylammoniumsulfonyl)carbamate). The molar ratio of the dehydrating agent to the compound of formula 18 may be 2:1 to 10:1, such as 4.6:1.

In step (a), the base may be an organic base, such as *N*,*N*-diisopropylethylamine and/or triethylamine. The molar ratio of the base to the compound of formula 18 may be 4:1 to 6.5:1, such as 4.1:1 or 6.2:1.

The ring-closing reaction is preferably conducted under an inert gas, such as nitrogen or argon.

The ring-closing reaction may be conducted at a temperature of 10 °C to 45 °C, such as 25 °C to 35 °C.

The progress of the ring-closing reaction may be detected by a conventional monitoring method in the art (e.g., TLC, HPLC, or NMR), and the end point of the reaction is generally considered the point at which the compound of formula 18 disappears or no longer reacts. For example, with the end point of the reaction considered the point at which the compound of formula 18 disappears or no longer reacts, the ring-closing reaction is preferably conducted for a period of 10 h to 30 h, such as 20 h.

Step (a) may also comprise a post-treatment, wherein the post-treatment may comprise the following steps: after the ring-closing reaction is completed, adding water to quench the reaction, and washing and separating the obtained organic phase. The quenching may be conducted by adding water in an amount of 5-10 times the mass of the compound of formula 18 with the temperature controlled at 15-25 °C; the washing may be conducted by adding a saturated aqueous sodium chloride solution in an amount of 5-10 times the mass of the compound of formula 18. In a certain embodiment of the present invention, preferably, the organic phase containing the compound of formula 20 after the post-treatment may be directly fed to the next step without purification.

In step (b), the organic solvent may be one or more of an aromatic hydrocarbon solvent, an ether solvent, an ester solvent, and a halogenated hydrocarbon solvent, wherein the aromatic hydrocarbon solvent may be toluene, the ether solvent may be tetrahydrofuran, the ester solvent may be ethyl acetate, and the halogenated hydrocarbon solvent may be dichloromethane. The volume ratio of the water to the organic solvent may be 1.3:1 to 1:1. The solvent may be used in an amount that does not affect the reaction, for example, the mass ratio of the water to the compound of formula 19 may be 5:1 to 10:1.

In step (b), the acidic condition may be pH 6-8; the acidic condition may be achieved by adding 5% citric acid for adjustment, wherein the 5% citric acid may be added at a temperature of 15-25 °C.

The tautomerization reaction may be conducted at a temperature of 10 °C to 45 °C, such as 35 °C to 45 °C.

The progress of the tautomerization reaction may be detected by a conventional monitoring method in the art (e.g., TLC, HPLC, or NMR), and the end point of the reaction is generally considered the point at which the compound of formula 19 disappears or no longer reacts. The tautomerization reaction is preferably conducted for a period of 10 h to 30 h, such as 20 h.

Step (b) may also comprise a post-treatment, wherein the post-treatment may comprise the following steps: after the tautomerization reaction is completed, adding *n*-heptane, stirring, cooling to 5-15 °C, stirring, filtering, rinsing with water, and drying. The *n*-heptane is preferably dropwise added, and the *n*-heptane may be added at a temperature of 15-25 °C; the mass ratio of the *n*-heptane to the compound of formula 20 may be 3:1; the stirring may be conducted for a period of 3 h; the mass ratio of the water for the rinsing to the compound of formula 20 may be 3: 1.

In step (b), the hydrazide compound of formula 19 may be prepared by step (a).

Step (a) may also comprise a method for preparing the hydrazide compound of formula 18 and/or the tautomer thereof, which comprises the following step (c):
in a solvent, subjecting an anhydride compound of formula 24 to an amidation reaction with a hydrazine compound of formula 17 and/or a tautomer thereof to give the hydrazide compound of formula 18 and/or the tautomer thereof:

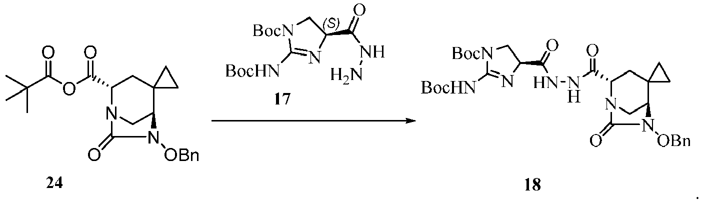

The solvent may be one or more of an aromatic hydrocarbon solvent, an ether solvent, an ester solvent, and a halogenated hydrocarbon solvent, wherein the aromatic hydrocarbon solvent may be toluene, the ether solvent may be tetrahydrofuran, the ester solvent may be ethyl acetate, and the halogenated hydrocarbon solvent may be dichloromethane. The solvent may be used in an amount that does not affect the reaction, for example, the volume-to-mass ratio of the solvent to the compound of formula 17 may be 10 mL/g to 20 mL/g.

The molar ratio of the anhydride compound of formula 24 to the hydrazine compound of formula 17 and/or the tautomer thereof may be 1.3:1 to 1:1.3, such as 1:1.2.

The amidation reaction may be conducted at a temperature of -15 °C to 0 °C, such as -10 °C to 0 °C.

Preferably, the method comprises the following step: with the temperature controlled at -10 to 0 °C, adding a mixture of the anhydride compound of formula 24 and the solvent to a mixture of the compound of formula 17 and the solvent to conduct the amidation reaction to give the hydrazide compound of formula 18 and/or the tautomer thereof.

The amidation reaction is preferably conducted under an inert gas, such as nitrogen or argon.

The progress of the amidation reaction may be detected by a conventional monitoring method in the art (e.g., TLC, HPLC, or NMR), and the end point of the reaction is generally considered the point at which the compound of formula 24 disappears or no longer reacts. The amidation reaction is preferably conducted for a period of 1 h to 3 h, such as 1 h.

Step (c) may also comprise a post-treatment, wherein the post-treatment may comprise the following steps: after the amidation reaction is completed, washing an organic phase, then replacing the organic solvent by ethyl acetate, washing with water, adding methyl *tert*-butyl ether and mixing for precipitation, filtering, washing, and drying; the washing may be conducted by sequentially using water and a 5% aqueous sodium bicarbonate solution, and the water and the 5% aqueous sodium bicarbonate solution may be used in an amount of 0.3 time the volume of the reaction system; the washing is preferably conducted at 10 °C; the replacement of the organic solvent may be conducted by the following steps: concentrating the washed organic phase (to 2-4 times the volume of the product), adding ethyl acetate (in an amount of 4-5 times the volume of the product) and mixing, concentrating (to about 4 times the volume of the product), then adding ethyl acetate (in an amount of 9-10 times the volume of the product) and water (in an amount of 2-3 times the volume of the product) and mixing, separating the obtained organic phase, and concentrating (to 4-6 times the volume of the product) to give a concentrate, which can be used for a crystallization step. The precipitation may be conducted by adding methyl *tert*-butyl ether (in an amount of 3-5 times the volume of the product), mixing, and cooling to precipitate a solid, wherein the mixing may be conducted at a temperature of 35-45 °C, and the cooling may be cooling to 10-20 °C; the solid may be washed with a mixed solution (in a volume ratio of 1.2:1) of ethyl acetate and methyl *tert*-butyl ether (in an amount of 0.8-1 times the volume of the product) after filtration.

The starting materials for the amidation reaction are the anhydride compound of formula 24, the hydrazine compound of formula 17 and/or the tautomer thereof, and the solvent.

In a certain embodiment, the method for preparing the hydrazide compound of formula 18 and/or the tautomer thereof may include the following scheme (i) and/or scheme (ii):
scheme (i) is a method for preparing the anhydride compound of formula 24, which comprises the following step (d): in a solvent, in the presence of a base, subjecting a carboxylic acid compound of formula 9 to an acylation reaction with pivaloyl chloride to give the anhydride compound of formula 24: 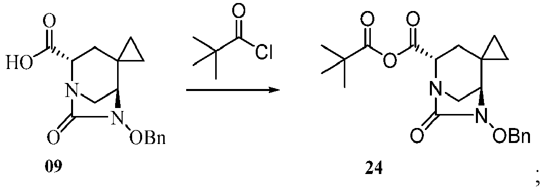
scheme (ii) is a method for preparing the hydrazine compound of formula 17, which comprises the following step (e): subjecting a hydrazine to a hydrazidation reaction as shown below with an azacycle compound of formula 16 and/or a tautomer thereof to give the hydrazide compound of formula 17 and/or the tautomer thereof:

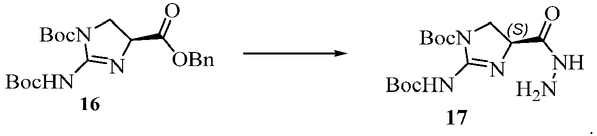

In step (d), the molar ratio of the carboxylic acid compound of formula 9 to pivaloyl chloride may be 1:1 to 1:1.6, such as 1:1.1.

The solvent may be one or more of an aromatic hydrocarbon solvent, an ether solvent, an ester solvent, and a halogenated hydrocarbon solvent, wherein the aromatic hydrocarbon solvent may be toluene, the ether solvent may be tetrahydrofuran, the ester solvent may be ethyl acetate, and the halogenated hydrocarbon solvent may be dichloromethane. The solvent may be used in an amount that does not affect the reaction, for example, the volume-to-mass ratio of the solvent to the compound of formula 9 may be 10 mL/g to 20 mL/g.

The base may be an organic base, such as one or more of *N*,*N*-diisopropylethylamine (DIPEA), pyridine, and triethylamine.

The molar ratio of the base to the carboxylic acid compound of formula 9 may be 3:1 to 1:1, such as 2:1.

The acylation reaction is preferably conducted under an inert gas, such as nitrogen or argon.

The acylation reaction may be conducted at a temperature of -10 to 10 °C, such as -10 to 0 °C.

Step (d) preferably comprises: sequentially adding the base and pivaloyl chloride to a mixture of the compound of formula 9 and the solvent at -5 °C or lower, and then conducting the acylation reaction at -10 to 0 °C to give the anhydride compound of formula 24.

The progress of the acylation reaction may be detected by a conventional monitoring method in the art (e.g., TLC, HPLC, or NMR), and the end point of the reaction is generally considered the point at which the compound of formula 9 disappears or no longer reacts. The amidation reaction is preferably conducted for a period of 1 h to 3 h, such as 2.5 h.

After the acylation reaction is completed, the amidation reaction may be directly conducted without a post-treatment.

In step (e), the molar ratio of the hydrazine to the azacycle compound of formula 16 and/or the tautomer thereof may be 1.5:1 to 3:1.

The hydrazine may be hydrazine hydrate, such as 80% hydrazine hydrate.

The solvent is one or more of an alcohol solvent, an aromatic hydrocarbon solvent, and a halogenated alkane, wherein the alcohol solvent may be one or more of methanol, ethanol, and isopropanol, the aromatic hydrocarbon solvent may be toluene, and the halogenated alkane may be dichloromethane. The solvent may be used in an amount that does not affect the reaction, for example, the volume-to-mass ratio of the solvent to the compound of formula 16 may be 10 mL/g to 20 mL/g, such as 12 mL/g.

The hydrazidation reaction is preferably conducted under an inert gas, such as nitrogen or argon.

The hydrazidation reaction may be conducted at a temperature of -20 to 10 °C, such as -10 to 5 °C.

Step (e) preferably comprises: with the temperature controlled at 5 °C or lower, adding the hydrazine to a mixture of the azacycle compound of formula 16 and the solvent to conduct the hydrazidation reaction to give the hydrazide compound of formula 17 and/or the tautomer thereof.

The progress of the hydrazidation reaction may be detected by a conventional monitoring method in the art (e.g., TLC, HPLC, or NMR), and the end point of the reaction is generally considered the point at which the compound of formula 16 disappears or no longer reacts. The hydrazidation reaction is preferably conducted for a period of 1 h to 3 h, such as 1 h.

Step (e) may also comprise a post-treatment, wherein the post-treatment may comprise the following steps: after the hydrazidation reaction is completed, sequentially adding an aqueous ammonium chloride solution (10%) and dichloromethane, mixing, separating, extracting an aqueous phase with dichloromethane, combining organic phases, washing with water, and concentrating to give a concentrate, which can be directly used for the next amidation reaction.

Scheme (ii) may also comprise a method for preparing the azacycle compound of formula 16 and/or the tautomer thereof, which comprises the following step (f):
in a solvent, in the presence of triphenylphosphine, imidazole and iodine, subjecting a guanidine compound of formula 15 to an Appel reaction and a ring-closing reaction as shown below to give the azacycle compound of formula 16 and/or the tautomer thereof:

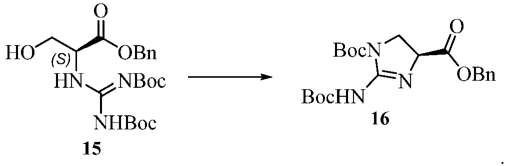

The molar ratio of the triphenylphosphine to the guanidine compound of formula 15 may be 1.1:1 to 1.3:1.

The molar ratio of the imidazole to the guanidine compound of formula 15 may be 2.2:1 to 2.6:1.

The molar ratio of the iodine to the guanidine compound of formula 15 may be 1.1:1 to 1.3:1.

The solvent may be a nitrile solvent and/or a halogenated hydrocarbon solvent, wherein the nitrile solvent may be acetonitrile, and the halogenated hydrocarbon solvent may be dichloromethane; the solvent may be used in an amount that does not affect the reaction, for example, the volume-to-mass ratio of the solvent to the compound of formula 15 may be 16 mL/g to 40 mL/g.

The Appel reaction and the ring-closing reaction may be conducted at a temperature of -10 °C to 10 °C, such as -5 °C to 5 °C.

The Appel reaction and the ring-closing reaction are preferably conducted under an inert gas, such as nitrogen or argon.

Step (f) preferably comprises: dropwise adding a mixture of the guanidine compound of formula 15 and the solvent to a mixture of the iodine, the triphenylphosphine, the imidazole and the solvent.

Step (f) preferably comprises: at -5 °C to 5 °C, adding the iodine to a mixture of the triphenylphosphine, the imidazole and the solvent in portions to give a reaction system 1, and then adding a mixture of the guanidine compound of formula 15 and the solvent to the above reaction system 1 to conduct the Appel reaction and the ring-closing reaction to give the azacycle compound of formula 16 and/or the tautomer thereof. The mixture of the guanidine compound of formula 15 and the solvent is preferably dropwise added to the reaction system 1.

The progress of the ring-closing reaction may be detected by a conventional monitoring method in the art (e.g., TLC, HPLC, or NMR), and the end point of the reaction is generally considered the point at which the compound of formula 15 disappears or no longer reacts. The Appel reaction and the ring-closing reaction are preferably conducted for a period of 1 h to 3 h, such as 1 h.

Step (f) may also comprise a post-treatment, wherein the post-treatment may comprise the following steps: after the ring-closing reaction is completed, adding a (5%) aqueous Na₂SO₃ solution, separating the obtained organic phase, washing with water, concentrating, adding DMF and *n*-heptane (in a mass ratio approximately equal to 14: 1) and mixing, concentrating, adding water and mixing, filtering, washing a filter cake with DMF/water (in a volume ratio approximately equal to 1:1), adding ethyl acetate for dissolution, adding water for washing (twice), concentrating, adding *n*-heptane and methyl *tert*-butyl ether (in a mass ratio approximately equal to 1:1) and mixing, filtering, washing a filter cake, and drying to give the azacycle compound of formula 16 and/or the tautomer thereof. For example, the (5%) aqueous Na₂SO₃ solution is added at -5 to 5 °C.

In scheme (ii), the guanidine compound of formula 15 may be prepared by the following step: in an organic solvent and water, in the presence of a base, subjecting a compound of formula 13 to an imidization reaction as shown below with a compound of formula 14 to give the guanidine compound of formula 15:

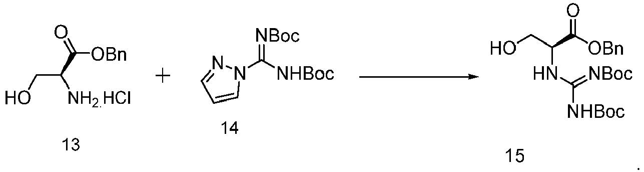

The molar ratio of the compound of formula 13 to the compound of formula 14 may be 1:1.

The organic solvent may be a nitrile solvent and/or a halogenated hydrocarbon solvent, wherein the nitrile solvent may be acetonitrile, and the halogenated hydrocarbon solvent may be dichloromethane. The solvent may be used in an amount that does not affect the reaction, for example, the volume-to-mass ratio of the organic solvent to the compound of formula 14 may be 6 mL/g to 15 mL/g, such as 8 mL/g to 10 mL/g.

The imidization reaction is preferably conducted under an inert gas, such as nitrogen or argon.

The imidization reaction may be conducted at a temperature of 10 °C to 40 °C, such as 20 °C to 30 °C.

The base may be an alkali metal carbonate and/or bicarbonate, such as potassium carbonate and/or potassium bicarbonate.

The mass ratio of the water to the base may be (1.5-2.5):1, such as 2:1.

The progress of the imidization reaction may be detected by a conventional monitoring method in the art (e.g., TLC, HPLC, or NMR), and the end point of the reaction is generally considered the point at which the compound of formula 15 disappears or no longer reacts. The imidization reaction is preferably conducted for a period of 10 h to 20 h, such as 16 h.

Step (f) may also comprise a post-treatment, wherein the post-treatment may comprise the following steps: after the imidization reaction is completed, adding water for washing an organic phase (e.g., twice), separating the obtained organic phase, and concentrating.

In the present invention, the tautomer means that compounds containing fragments 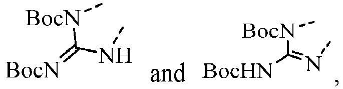 respectively, are tautomers of each other, or that compounds containing fragments 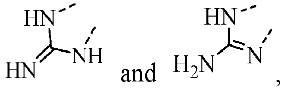 respectively, are tautomers of each other.

The present invention provides an azacycle compound of formula 16 and/or a tautomer thereof, a hydrazine compound of formula 17 and/or a tautomer thereof, an anhydride compound of formula 24, or a hydrazide compound of formula 18 and/or a tautomer thereof: 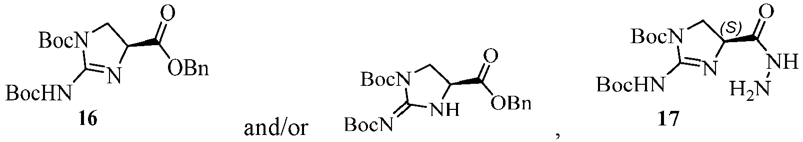 and/or 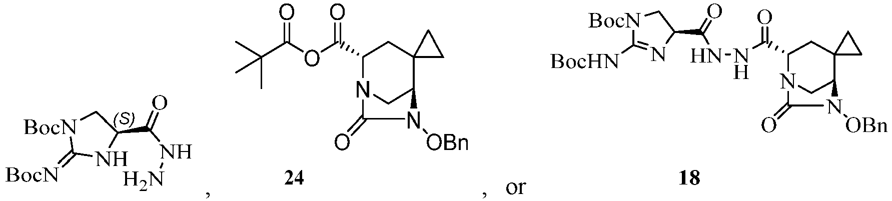 and/or

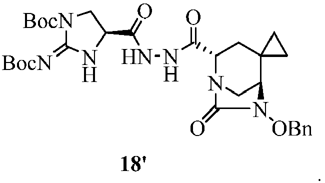

The present invention provides a preparation method for a hydrazide compound of formula 18 and/or a tautomer thereof, comprising the following step:
in a solvent, subjecting an anhydride compound of formula 24 to an amidation reaction with a hydrazine compound of formula 17 and/or a tautomer thereof to give the hydrazide compound of formula 18 and/or the tautomer thereof:

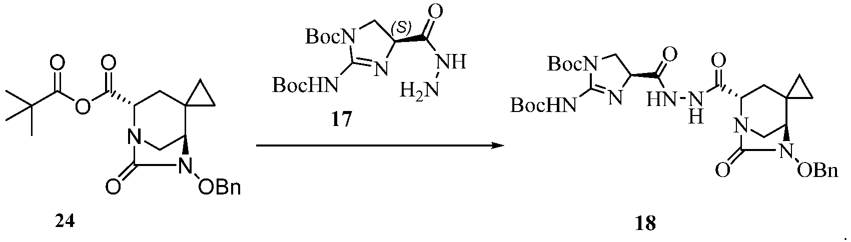

The operations and reaction conditions in the preparation method for the hydrazide compound of formula 18 and/or the tautomer thereof may be the same as those in the method for preparing the hydrazide compound of formula 18 and/or the tautomer thereof in the preparation method for the oxadiazazole compound and/or the tautomer thereof described above.

The present invention provides a preparation method for an anhydride compound of formula 24, comprising the following step: in a solvent, in the presence of a base, subjecting a carboxylic acid compound of formula 9 to an acylation reaction with pivaloyl chloride to give the anhydride compound of formula 24:

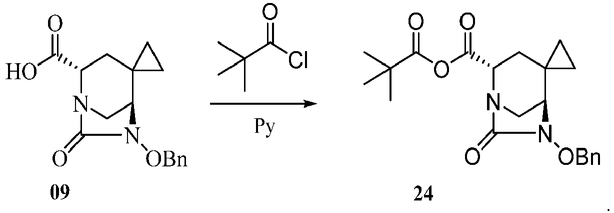

The operations and reaction conditions in the preparation method for the anhydride compound of formula 24 and/or the tautomer thereof may be the same as those in the method for preparing the anhydride compound of formula 24 and/or the tautomer thereof in the preparation method for the oxadiazazole compound and/or the tautomer thereof described above.

The present invention provides a preparation method for the hydrazine compound of formula 17 and/or the tautomer thereof, comprising the following step:

subjecting a hydrazine to a hydrazidation reaction as shown below with an azacycle compound of formula 16 and/or a tautomer thereof to give the hydrazide compound of formula 17 and/or the tautomer thereof:

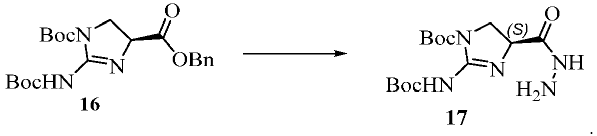

The operations and reaction conditions in the preparation method for the hydrazine compound of formula 17 and/or the tautomer thereof may be the same as those in the method for preparing the hydrazide compound of formula 17 and/or the tautomer thereof in the preparation method for the oxadiazazole compound and/or the tautomer thereof described above.

The present invention provides a preparation method for the azacycle compound of formula 16 and/or the tautomer thereof, comprising the following step:
in a solvent, in the presence of triphenylphosphine, imidazole and iodine, subjecting a guanidine compound of formula 15 to an Appel reaction and a ring-closing reaction as shown below to give the azacycle compound of formula 16 and/or the tautomer thereof:

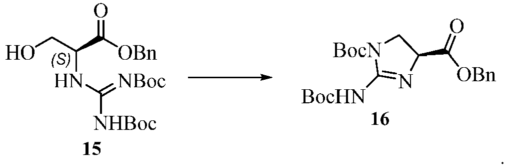

The reaction conditions and operations in the preparation method for the azacycle compound of formula 16 and/or the tautomer thereof may be the same as those in the method for preparing the azacycle compound of formula 16 and/or the tautomer thereof in the preparation method for the oxadiazazole compound and/or the tautomer thereof described above.

The present invention provides a preparation method for a β-lactamase inhibitor and an intermediate, being the following scheme:
scheme 1, a preparation method for a hydroxylamine compound of formula 21 and/or a tautomer thereof, which comprises the following steps:
   step (1), comprising the following step (a) and/or step (b):
      step (a):
         in a solvent, in the presence of a dehydrating agent and a base, subjecting a hydrazide compound of formula 18 and/or a tautomer thereof to a ring-closing reaction as shown to give an oxadiazazole compound of formula 19 and/or a tautomer thereof: 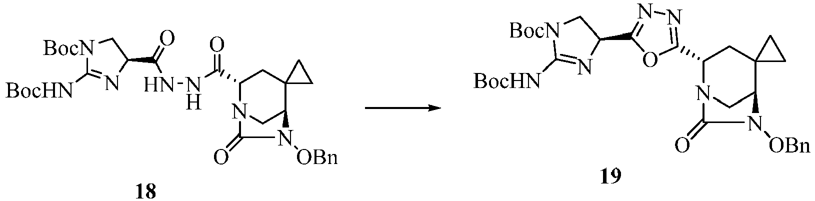
      step (b): in a solvent, under an acidic condition, subjecting a hydrazide compound of formula 19 to a tautomerization reaction as shown to give a tautomer thereof, namely an oxadiazazole compound of formula 20: 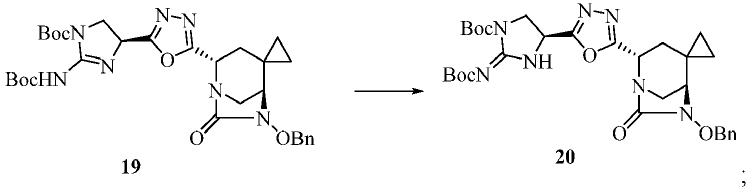 and
   step (2): in a solvent, in the presence of a palladium catalyst and hydrogen, subjecting the imidazoline compound of formula 20 and/or a tautomer thereof to a debenzylation reaction as shown to give the hydroxylamine compound of formula 21 and/or the tautomer thereof, wherein the preparation of the imidazoline compound of formula 20 and/or the tautomer thereof is the same as that in step (a) or (b) described above: 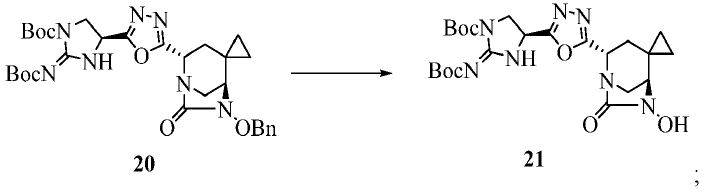
scheme 2, a preparation method for a hydroxylamine compound of formula 21 and/or a tautomer thereof, which comprises the following steps:
   step (1): the same as step (1) in scheme 1;
   step (2): the same as step (2) in scheme 1; and
   step (3): in a solvent, in the presence of pyridine and a sulfonation reagent, subjecting the hydroxylamine compound of formula 21 and/or the tautomer thereof to a sulfonation reaction as shown to give a sulfonic acid oxygen compound of formula 22 and/or a tautomer thereof: 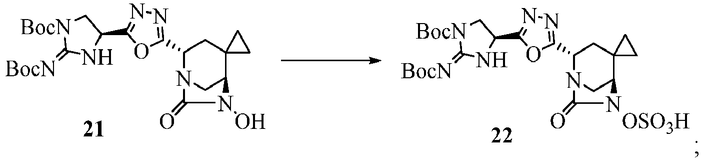 or
scheme 3, a preparation method for an imine compound of formula I, which comprises the following steps:
   steps (1) to (3): the same as steps (1) to (3) in scheme 2; and
   step (4): in water and an organic solvent, subjecting the sulfonic acid oxygen compound of formula 22 and/or the tautomer thereof to an amine protecting group-removing reaction as shown to give the imine compound of formula I:

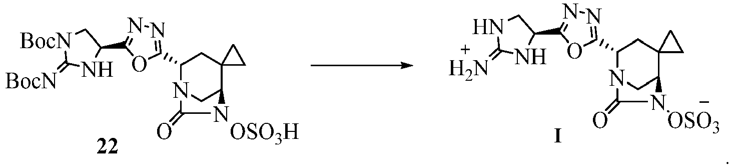

In step (1) of scheme 1, the reaction operations and conditions are the same as those in the preparation method for the oxadiazazole compound and/or the tautomer thereof described above.

In step (2) of scheme 1, the solvent may be one or more of an alcohol solvent, an ether solvent, an amide solvent, and a halogenated hydrocarbon solvent, wherein the alcohol solvent may be one or more of methanol, ethanol, and isopropanol, the halogenated hydrocarbon solvent may be dichloromethane, the ether solvent may be tetrahydrofuran, and the amide solvent may be dimethylacetamide (DMAc) and/or *N*-methyl-2-pyrrolidone (NMP); for example, the solvent may be tetrahydrofuran. The solvent may be used in an amount that does not affect the reaction, for example, the volume-to-mass ratio of the solvent to the imidazoline compound of formula 20 and/or the tautomer thereof may be 15 mL/g to 27 mL/g.

The palladium catalyst may be palladium on carbon or palladium hydroxide, such as 10% palladium on carbon (dry basis).

The mass ratio of the palladium catalyst to the imidazoline compound of formula 20 and/or the tautomer thereof may be 0.02: 1 to 0.9:1, such as 0.09: 1.

The pressure of the hydrogen may be 0.3 Mpa to 0.5 Mpa.

The debenzylation reaction may be conducted at a temperature of 0 °C to 40 °C, such as 10 °C to 20 °C.

The progress of the debenzylation reaction may be detected by a conventional monitoring method in the art (e.g., TLC, HPLC, or NMR), and the end point of the reaction is generally considered the point at which the imidazoline compound of formula 20 and/or the tautomer thereof disappears or no longer reacts. The debenzylation reaction is preferably conducted for a period of 10 h to 30 h, such as 24 h.

Scheme 1 may also comprise a post-treatment, wherein the post-treatment may comprise the following steps: after the debenzylation reaction is completed, adding dimethyl sulfoxide, mixing, filtering, washing a filter cake with tetrahydrofuran, combining filtrates, adding mercaptoalkyl functionalized silica and activated carbon (in a mass ratio of 1:1), mixing, filtering, washing a filter cake with tetrahydrofuran, combining filtrates, concentrating, adding water and acetonitrile (in a mass ratio of 1.27:1), mixing, filtering, rinsing, and drying. The amount of the dimethyl sulfoxide used may be 10 to 12 times that of the imidazoline compound of formula 20 and/or the tautomer thereof; the amount of the mercaptoalkyl functionalized silica and activated carbon used may be 0.16 times that of the imidazoline compound of formula 20 and/or the tautomer thereof; the amount of the water and acetonitrile used may be 14 to 15 times that of the imidazoline compound of formula 20 and/or the tautomer thereof.

In step (3) of scheme 2, the solvent may be a halogenated hydrocarbon solvent and/or a nitrile solvent, wherein the halogenated hydrocarbon solvent may be dichloromethane, and the nitrile solvent may be acetonitrile; for example, the solvent may be acetonitrile. The solvent may be used in an amount that does not affect the reaction, for example, the volume-to-mass ratio of the solvent to the hydroxylamine compound of formula 21 and/or the tautomer thereof may be 5 mL/g to 20 mL/g.

The sulfonation reagent may be one or more of sulfur trioxide pyridine, sulfur trioxide triethylamine, and sulfur trioxide trimethylamine.

The mass ratio of the sulfur trioxide pyridine to the hydroxylamine compound of formula 21 and/or the tautomer thereof may be 1.2:1 to 6:1, such as 1.2:1 to 5.97:1.

The mass ratio of the pyridine to the hydroxylamine compound of formula 21 and/or the tautomer thereof may be 2.5:1 to 6.25:1.

The sulfonation reaction may be conducted at a temperature of 15 °C to 35 °C, such as 25 °C to 35 °C.

The progress of the sulfonation reaction may be detected by a conventional monitoring method in the art (e.g., TLC, HPLC, or NMR), and the end point of the reaction is generally considered the point at which the hydroxylamine compound of formula 21 and/or the tautomer thereof disappears or no longer reacts. The sulfonation reaction is preferably conducted for a period of 5 h to 10 h, such as 6 h.

Step (3) of scheme 2 may also comprise a post-treatment, wherein the post-treatment may comprise the following steps: after the sulfonation reaction is completed, adding activated carbon, mixing, filtering, washing a filter cake with acetonitrile, and combining filtrates. The filtrate can be directly used for the next step.

In step (4) of scheme 3, the organic solvent may be a nitrile solvent, wherein the nitrile solvent may be acetonitrile. The water and the organic solvent may be used in an amount that does not affect the reaction, for example, the volume-to-mass ratio of the organic solvent to the sulfonic acid oxygen compound of formula 22 and/or the tautomer thereof may be 9 mL/g to 11 mL/g.

The amine protecting group-removing reaction may be conducted at a temperature of 18 °C to 40 °C, such as 30 °C to 40 °C.

The progress of the amine protecting group-removing reaction may be detected by a conventional monitoring method in the art (e.g., TLC, HPLC, or NMR), and the end point of the reaction is generally considered the point at which the sulfonic acid oxygen compound of formula 22 and/or the tautomer thereof disappears or no longer reacts. The amine protecting group-removing reaction is preferably conducted for a period of 3 h to 10 h, such as 5 h.

Step (4) of scheme 3 may also comprise the following purification steps: after the reaction is completed, cooling to 0 °C to 10 °C to precipitate a solid, filtering, washing a filter cake with a 10% aqueous acetonitrile solution, adding dimethyl sulfoxide, heating to 45-65 °C, mixing, dropwise adding acetonitrile at this temperature, cooling to 0-10 °C and mixing to precipitate a solid, filtering, rinsing a filter cake with acetonitrile, then adding water, controlling the temperature at 20-30 °C, mixing, cooling to 0-10 °C and mixing to precipitate a solid, filtering, washing a filter cake with water, and drying.

The present invention provides a preparation method for a β-lactamase inhibitor, comprising the following steps: 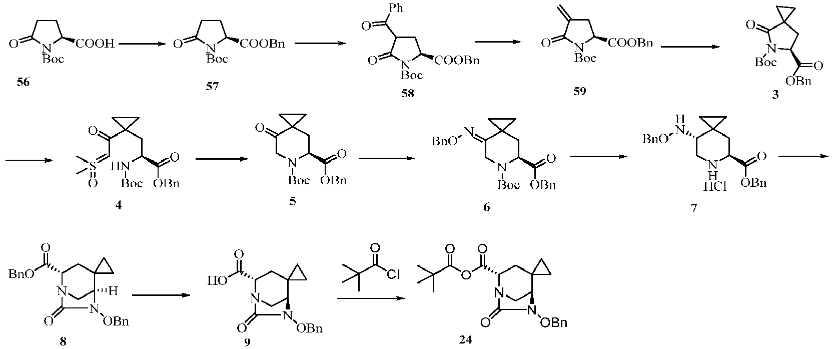 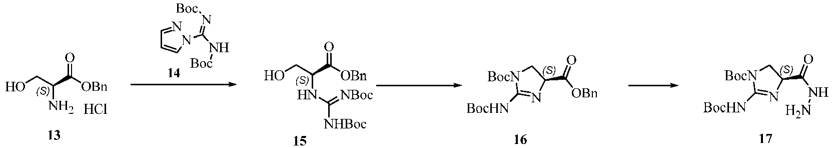 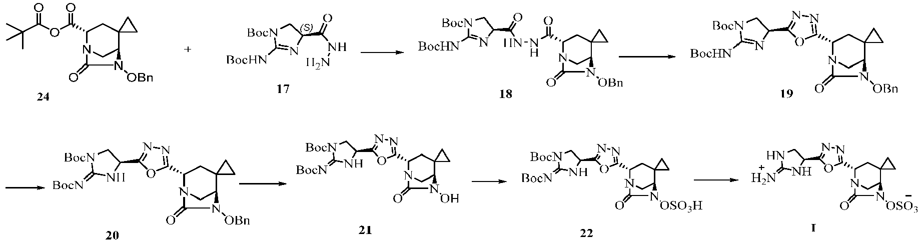
step 1: in a solvent, in the presence of a base and a phase transfer catalyst, subjecting a compound of formula 56 to a benzyl esterification reaction as shown below with benzyl bromide to give a compound of formula 57;
step 2: in a solvent, in the presence of a base, subjecting the compound of formula 57 to a benzoylation reaction as shown below with benzoyl chloride to give a compound of formula 58;
step 3: in a solvent, in the presence of a base, an aqueous formaldehyde solution or paraformaldehyde, and a phase transfer catalyst, subjecting the compound of formula 58 to a reaction as shown below to give a compound of formula 59;
step 4: in a solvent, in the presence of a Zn/Cu or Zn/Ag reagent, subjecting the compound of formula 59 to a cyclopropanation reaction as shown below with a methylating reagent to give a compound of formula 3;
step 5: in a solvent, in the presence of a base, subjecting the compound of formula 3 to a ring-opening reaction as shown below with trimethylsulfoxonium iodide to give compound 4;
step 6: in a solvent, in the presence of a catalyst and a ligand, subjecting the compound of formula 4 to a ring-closing reaction as shown below to give compound 5;
step 7: in a solvent, in the presence of a base, subjecting the compound of formula 5 to a Schiff base reaction with *O*-phenylhydroxylamine or a salt thereof to give compound 6;
step 8: (1) in a solvent, in the presence of sulfuric acid, reacting the compound of formula 6 with an acid to give a mixture A; and (2) adding a reducing agent to the mixture A to conduct a reduction reaction to give a compound of formula 7;
step 9: adding a mixture of triphosgene and a solvent to a mixture of the compound of formula 7 and/or a salt thereof, a base and a solvent to conduct an amidation ring-closing reaction as shown below to give a diazabicyclooctane compound of formula 8;
step 10: in an organic solvent and water, adding a base to the compound of formula 8 for hydrolysis to give a compound of formula 9;
step 11: in a solvent, in the presence of a base, subjecting the carboxylic acid compound of formula 9 to an acylation reaction with pivaloyl chloride to give an anhydride compound of formula 24;
step 12: in an organic solvent and water, in the presence of a base, subjecting a compound of formula 13 to an imidization reaction as shown below with a compound of formula 14 to give a guanidine compound of formula 15;
step 13: in a solvent, in the presence of triphenylphosphine, imidazole and iodine, subjecting the guanidine compound of formula 15 to an Appel reaction and a ring-closing reaction as shown below to give an azacycle compound of formula 16 and/or a tautomer thereof;
step 14: subjecting a hydrazine to a hydrazidation reaction as shown below with the azacycle compound of formula 16 and/or the tautomer thereof to give a hydrazide compound of formula 17 and/or a tautomer thereof;
step 15: subjecting the anhydride compound of formula 24 to an amidation reaction with the hydrazine compound of formula 17 and/or the tautomer thereof to give a hydrazide compound of formula 18 and/or a tautomer thereof;
step 16: in a solvent, in the presence of a dehydrating agent and a base, subjecting the hydrazide compound of formula 18 and/or the tautomer thereof to a ring-closing reaction as shown to give an oxadiazazole compound of formula 19 and/or a tautomer thereof;
step 17: in an organic solvent and water, under an acidic condition, subjecting the hydrazide compound of formula 19 to a tautomerization reaction as shown to give a tautomer thereof, namely an oxadiazazole compound of formula 20;
step 18: in a solvent, in the presence of a palladium catalyst and hydrogen, subjecting the imidazoline compound of formula 20 and/or a tautomer thereof to a debenzylation reaction as shown to give a hydroxylamine compound of formula 21 and/or a tautomer thereof;
step 19: in a solvent, in the presence of pyridine and a sulfonation reagent, subjecting the hydroxylamine compound of formula 21 and/or the tautomer thereof to a sulfonation reaction as shown to give a sulfonic acid oxygen compound of formula 22 and/or a tautomer thereof; and
step 20: in water and an organic solvent, subjecting the sulfonic acid oxygen compound of formula 22 and/or the tautomer thereof to an amine protecting group-removing reaction as shown to give an imine compound of formula I.

The operations and conditions for each reaction in the preparation method may be the same as those for the corresponding reaction in any of the schemes described above in the present invention.

The above preferred conditions may be combined arbitrarily to obtain preferred embodiments of the present invention without departing from the general knowledge in the art.

The reagents and starting materials used in the present invention are commercially available.

The positive and progressive effects of the present invention are as follows: the method for preparing the β-lactamase inhibitor and the intermediate provided by the present invention has a low cost, improves the repeatability and scalability of key reactions, significantly increases the overall yield and reduces the number of reaction steps compared with the prior art, and has the technical advantages of being green, safe, efficient, simple, and convenient, so that the method is suitable for industrial production.

### DETAILED DESCRIPTION

The present invention is further illustrated by the following examples, which are not intended to limit the present invention. Experimental procedures without specified conditions in the following examples were performed in accordance with conventional procedures and conditions, or in accordance with instructions.

### Synthesis of XNW210003

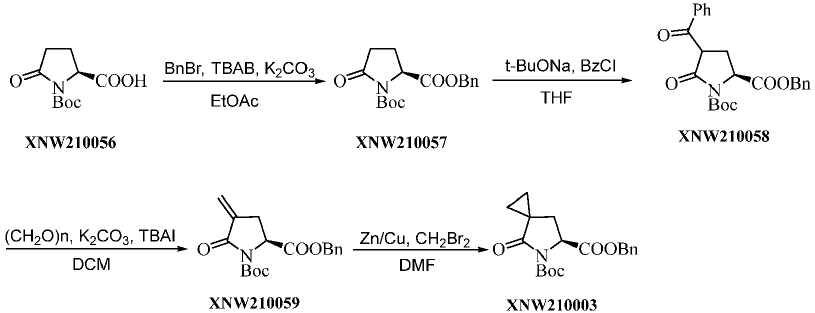

### Example 1

### Preparation of XNW210057

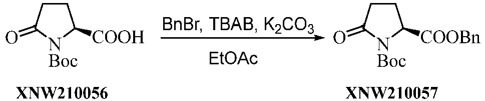

A reaction kettle was purged with nitrogen, and then 1000 mL of ethyl acetate, 100 g of XNW210056, 66.3 g of K₂CO₃, and 10 g of TBAB were sequentially added. The internal temperature was adjusted to 20-30 °C, and 74.6 g of BnBr was dropwise added at this temperature. The mixture was then heated to 45-55 °C and allowed to react at this temperature for 7 h. Sampling was performed under intermediate control until the reaction was completed, with a conversion rate of 99.1%.

The system was cooled to 15-25 °C and filtered. 500 mL of a 5% sodium chloride solution was added to the filtrate, and the mixture was stirred and left to stand for layer separation. With the temperature controlled below 50 °C, the organic phase was concentrated under reduced pressure until no liquid dripped out. 300 mL of *n*-heptane was added, and the organic phase was further concentrated under reduced pressure at a temperature below 50 °C until no liquid dripped out. Another 300 mL of *n*-heptane was added, and the organic phase was further concentrated under reduced pressure at a temperature below 50 °C until no liquid dripped out. 300 mL of *n*-heptane was then added, and the mixture was stirred at 15-25 °C for 2 h and filtered. The filter cake was then washed with 100 mL of *n*-heptane. The wet product was dried under vacuum at 50 °C to give 128 g of XNW210057 as a white solid, with a purity of 96.0%, an ee value of 99.76%, a content of 98%, and a yield of 91%.

### Example 2

### Preparation of XNW210058

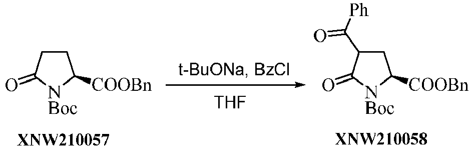

A reaction kettle was purged with nitrogen, and then 250 mL of tetrahydrofuran and 51 g (1.0 eq.) of XNW210057 were sequentially added. The internal temperature was adjusted to -15 to -5 °C, and 23.1 g (1.05 eq.) of benzoyl chloride was dropwise added at this temperature. 37.6 g (2.5 eq.) of sodium *tert*-butoxide was added in ten portions, and the mixture was allowed to react at this temperature for 1 h. Sampling was performed under intermediate control until the reaction was completed.

The temperature of the system was adjusted to -5 to 5 °C, and 250 mL of a 10% citric acid solution was added. The mixture was stirred and left to stand for layer separation, and the organic phase was concentrated under reduced pressure until no liquid dripped out. 250 mL of ethanol was added, and the organic phase was further concentrated under reduced pressure until no liquid dripped out. Another 250 mL of ethanol was added, and the organic phase was further concentrated under reduced pressure until no liquid dripped out. 250 mL of ethanol and 250 mL of water were then added, and the mixture was stirred at 20-25 °C for 1 h and filtered. The wet product was dried under vacuum to give 46 g of XNW210058 as a white solid, with a purity of 92.9% and a yield of 70%.

### Example 3

### Preparation of XNW210059 (1)

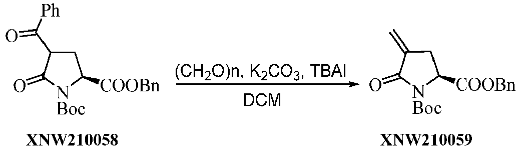

A reaction kettle was purged with nitrogen, and then 200 mL of dichloromethane, 21 g of XNW210058, 1.5 g of paraformaldehyde, 6.9 g of potassium carbonate, and 0.8 g of TBAI were sequentially added. The internal temperature was adjusted to 35-45 °C, and the mixture was allowed to react at this temperature for 2 h. 1.5 g of paraformaldehyde and 6.9 g of potassium carbonate were then added, and the mixture was allowed to react for 2 h. Another 1.5 g of paraformaldehyde and 6.9 g of potassium carbonate were then added, and the mixture was allowed to react for 11 h. Sampling was performed under intermediate control until the reaction was completed.

The temperature of the system was adjusted to 20-25 °C, and 150 mL of a 5% ammonium chloride solution was added. The mixture was stirred and left to stand for layer separation, and the organic phase was washed with 100 mL of water, and then concentrated under reduced pressure until no liquid dripped out to give 18 g of a crude product of XNW210059 as an oil, with a purity of 69.6%, a content of 56.1%, and a yield of 70%.

### Preparation of XNW210059 (2)

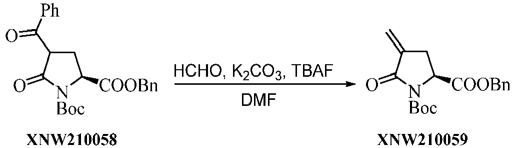

A reaction kettle was purged with nitrogen, and then 5 mL of *N*,*N*-dimethylformamide, 0.5 g of XNW210058, 0.14 g of an aqueous formaldehyde solution, 0.16 g of potassium carbonate, and 0.19 g of tetrabutylammonium fluoride trihydrate were sequentially added. The internal temperature was adjusted to 35 to 45 °C, and the mixture was allowed to react at this temperature for 3 h. Sampling was performed under intermediate control until the reaction was completed.

The temperature of the system was adjusted to 20-25 °C, and 10 mL of methyl *tert-*butyl ether and 10 mL of water were added. The mixture was stirred and left to stand for layer separation, and the aqueous phase was extracted once with 10 mL of methyl *tert*-butyl ether. The organic phases were combined, and then concentrated under reduced pressure until no liquid dripped out to give 0.36 g of a crude product of XNW210059 as an oil, with a purity of 85% and a yield of 80%.

### Example 4

### Preparation of XNW210003 (1)

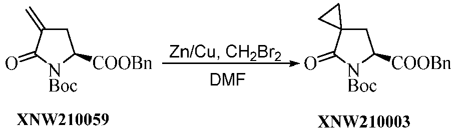

A reaction kettle was purged with nitrogen, and then 30 mL of DMF, 3 g (1.0 eq.) of XNW210059, 1.05 g (2.0 eq.) of Zn/Cu, and 2.8 g (2.0 eq.) of dibromomethane were sequentially added. The internal temperature was adjusted to 30-40 °C, and the mixture was allowed to react at this temperature for 17 h. Sampling was performed under intermediate control until the reaction was completed.

The temperature of the system was adjusted to 25 °C, and 30 mL of ethyl acetate was added, followed by dropwise addition of 30 mL of a 5% ammonium chloride solution. The mixture was stirred, filtered through celite, and left to stand for layer separation, and the aqueous phase was washed with 30 mL of ethyl acetate. The organic phases were combined, washed with 15 mL of 5% sodium chloride, and then concentrated under reduced pressure until no liquid dripped out. 18 mL of methanol and 9 mL of water were then added, and the mixture was stirred and filtered to give 4.8 g of a crude wet product of XNW210003, with a purity of 82.3%, a content of 41.1%, and a yield of 70%. ¹H NMR, CDCl₃, 7.37-7.26 (m, 5H), 5.22-5.21 (m, 2H), 4.74-4.69 (m, 1H), 2.56-2.49 (m, 1H), 1.93-1.87 (m, 1H), 1.45 (s, 9H), 1.32-1.15 (m, 2H), 0.80-0.72 (m, 2H), 1.28-1.21 (m, 2H), 1.07-0.88 (m, 2H), 0.71 (s, 2H), M/S: (m/z = 713.3, 2M+Na).

### Preparation of XNW210003 (2)

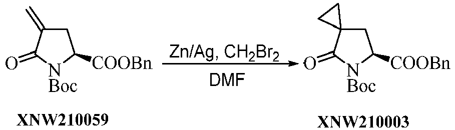

A reaction kettle was purged with nitrogen, and then 30 mL of DMF, 2.96 g (5.0 eq.) of Zn/Ag, and 7 g (5.0 eq.) of dibromomethane were sequentially added. The internal temperature was adjusted to 20-30 °C, and 3 g (1.0 eq.) of a solution of XNW210059 in DMF (15 mL) was dropwise added. The mixture was allowed to react at this temperature for 6 h, and sampling was performed under intermediate control until the reaction was completed.

The mixture was then filtered through celite, and the filter cake was rinsed with 30 mL of ethyl acetate, followed by dropwise addition of 45 mL of water. The mixture was stirred and left to stand for layer separation, and the organic phase was washed with 15 mL of water and then concentrated under reduced pressure until no liquid dripped out. 18 mL of methanol and 9 mL of water were then added, and the mixture was stirred and filtered. The wet product was dried to give 2.19 g of XNW210003, with a yield of 70%. ¹H NMR, CDCl₃, 7.37-7.26 (m, 5H), 5.22-5.21 (m, 2H), 4.74-4.69 (m, 1H), 2.56-2.49 (m, 1H), 1.93-1.87 (m, 1H), 1.45 (s, 9H), 1.32-1.15 (m, 2H), 0.80-0.72 (m, 2H), 1.28-1.21 (m, 2H), 1.07-0.88 (m, 2H), 0.71 (s, 2H), M/S: (m/z = 713.3, 2M+Na).

Preparation of Zn/Ag reagent: 50 mL of acetic acid was added to a reaction kettle under nitrogen, and the mixture was stirred, followed by addition of 50 mg of silver acetate. The system was heated to reflux, and 5.58 g of zinc powder was added. The system was then cooled to 20-30 °C and filtered, and the filter cake was added to the reaction kettle, followed by addition of 50 mL of tetrahydrofuran. The mixture was stirred for 30 min and filtered, and the filter cake was added to the reaction kettle, followed by addition of 50 mL of tetrahydrofuran. The mixture was stirred for 30 min and filtered, and the filter cake was dried to give 5.2 g of a Zn/Ag reagent.

### Synthesis of XNW210009

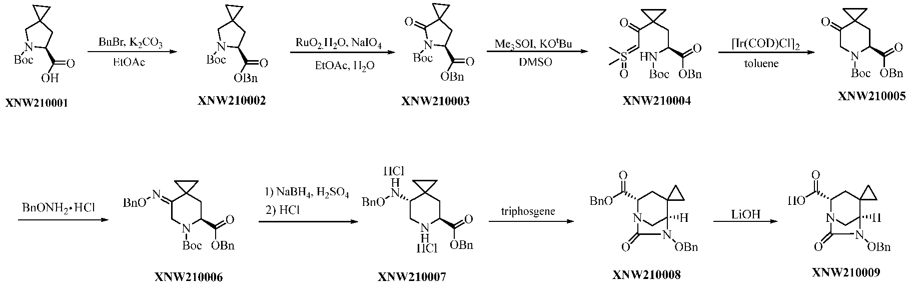

### Example 5

### Preparation of XNW210002

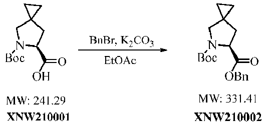

A reaction kettle was purged with nitrogen, and then 30 mL of ethyl acetate, 10 g (1.0 eq.) of XNW210001, and 6.3 g (1.1 eq.) of potassium carbonate were sequentially added. 7.1 g of benzyl bromide (1.0 eq.) was dropwise added with the temperature controlled at 30 °C or lower. The internal temperature was adjusted to 47-58 °C, and the mixture was stirred for 24 h or longer. Sampling was performed under intermediate control until the reaction was completed.

The reaction solution was cooled to 10-30 °C, and 30 g of water was added to the reaction solution. The mixture was stirred and left to stand for layer separation, and the organic phase was collected to give 36 g of a solution of XNW210002 in ethyl acetate, with a purity of 97.5%, a content of 35.6%, and a yield of 93.4%. ¹H NMR, DMSO-*d₆*, 7.38-7.37 (m, 5H), 5.22-5.06 (m, 2H), 4.41-4.33 (m, 1H), 3.36-3.28 (m, 1H), 3.22-3.15 (m, 1H), 2.36-2.29 (m, 1H), 1.70-1.60 (m, 1H), 1.39-1.28(m, 9H), 0.54-0.34(m, 4H), M/S: (m/z = 685.3, 2M+Na).

### Example 6

### Preparation of XNW210003

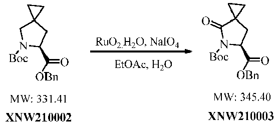

A reaction kettle was purged with nitrogen, and then 500 mL of water, 0.45 g (0.01 eq.) of ruthenium oxide monohydrate, 250 g (1.0 eq.) of a solution of XNW210002 in ethyl acetate (with a content of 40%), and 200 mL of ethyl acetate were sequentially added. 177.5 g (0.27 eq.) of sodium periodate was added in portions with the temperature controlled at 15-25 °C, and the mixture was allowed to react for 30 min. Sampling was performed under intermediate control until the reaction was completed.

30 g of celite was added to the reaction solution, and the mixture was stirred for another 30 min. The reaction solution was filtered through celite, and the filter cake was washed with 100 mL of ethyl acetate. The filtrate was stirred for 30 min and left to stand for layer separation, and the organic phase was collected. 500 mL of ethyl acetate was then added to the filter cake. The mixture was stirred for 30 min and filtered, and the filtrate was collected. The above organic phase and filtrate were combined, and then 544.4 g of a 10% aqueous sodium sulfite solution was added for washing. The organic phase was concentrated under reduced pressure to 1-1.5 times the volume with the temperature controlled at 50 °C or lower, and then 400 mL of *n*-heptane was added. The mixture was further concentrated under reduced pressure to 1-1.5 times the volume, and another 300 mL of *n*-heptane was added. The mixture was further concentrated under reduced pressure to 2-3 times the volume, then stirred at 10-30 °C for 2 h, and filtered. The filter cake was rinsed with 50 mL of *n*-heptane, and the wet product was dried at 50 °C under vacuum to give 88.2 g of XWN210003 as a white solid, with a content of 98.4%, a purity of 97%, and a yield of 83%. ¹H NMR, CDCl₃, 7.37-7.26 (m, 5H), 5.22-5.21 (m, 2H), 4.74-4.69 (m, 1H), 2.56-2.49 (m, 1H), 1.93-1.87 (m, 1H), 1.45 (s, 9H), 1.32-1.15 (m, 2H), 0.80-0.72 (m, 2H), 1.28-1.21 (m, 2H), 1.07-0.88 (m, 2H), 0.71 (s, 2H), M/S: (m/z = 713.3, 2M+Na).

### Example 7

### Preparation of XNW210004

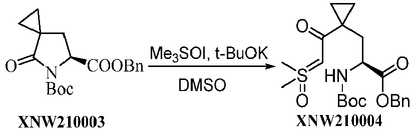

A reaction kettle was purged with nitrogen, and then 1000 mL of tetrahydrofuran, 151 g (1.2 eq.) of trimethylsulfoxonium iodide, 73.7 g (1.15 eq.) of potassium *tert*-butoxide, and 1200 mL of dimethyl sulfoxide were sequentially added. The internal temperature was adjusted to 20-30 °C, and the mixture was stirred for 2 h for later use.

Another reaction kettle was purged with nitrogen, and then 1000 mL of tetrahydrofuran and 200 g (1.0 eq.) of XNW210003 were sequentially added. The internal temperature was adjusted to -10 to 0 °C, and the above-prepared solution was dropwise added at this temperature. The mixture was allowed to react for 12 h, and sampling was performed under intermediate control until the reaction was completed.

1000 mL of toluene was added to the reaction solution, and with the temperature controlled at 15 °C or lower, 1000 mL of a 5% ammonium chloride solution was added. The mixture was stirred for 30 min with the temperature controlled at 15-25 °C and then left to stand for layer separation. The aqueous phase was washed with 600 mL of toluene, and the organic phases were combined, washed with 1000 mL of a 5% sodium chloride solution, then washed with 1000 mL of a 2% sodium chloride solution, and dried over 300 g of anhydrous magnesium sulfate. The reaction solution was filtered through celite, and the filter cake was washed with 600 mL of toluene. The filtrates were combined, and then concentrated under reduced pressure to 7-9 V with the jacket temperature controlled below 40 °C, thus giving 1366.5 g of a solution of XNW210004 in toluene, with a purity of 83%, a content of 13.7%, and a yield of 75%. ¹H NMR, CDCl₃, 7.37(s, 5H), 6.01-5.98 (m, 1H), 5.21-5.11 (m, 2H), 4.42-4.15 (m, 2H), 3.35-3.34 (m, 6H), 2.37-2.31 (m, 1H), 1.74-1.64 (m, 2H), 1.43 (s, 9H), 1.28-1.21 (m, 2H), 1.07-0.88 (m, 2H), 0.71 (s, 2H), M/S: (m/z = 438.2, M+H).

### Example 8

### Preparation of XNW210005

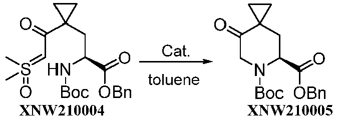

A reaction kettle was purged with nitrogen, and then 100 mL of toluene, 0.25 g (0.04 eq.) of triphenylphosphine, and 0.25 g (0.014 eq.) of chloro(cyclopentadienyl)bis(triphenylphosphine)ruthenium(II) were sequentially added. The internal temperature was adjusted to 95-105 °C, and 42 g (1.0 eq.) of a solution of XNW210004 in toluene was added at this temperature. The mixture was allowed to react at this temperature for 2 h, and sampling was performed under intermediate control until the reaction was completed.

The temperature of the system was adjusted to 20-30 °C, and 50 mL of water was added. The mixture was stirred and left to stand for layer separation, and the organic phase was concentrated under reduced pressure until no liquid dripped out. 80 mL of ethanol was added, and the organic phase was concentrated under reduced pressure until no liquid dripped out. Another 80 mL of ethanol was then added, and the organic phase was further concentrated under reduced pressure until no liquid dripped out, thus giving 38.5 g of a solution of XNW210004 in ethanol, with a purity of 75.7%, a content of 18.1%, and a yield of 85%. ¹H NMR, CDCl₃, 7.38-7.37 (m, 5H), 5.30-5.10 (m, 2H), 5.00-4.69 (m, 1H), 4.32-4.11 (m, 2H), 2.43-2.31 (m, 1H), 2.04-1.98 (m, 1H), 1.59 (s, 2H), 1.49-1.40 (m, 10H), 1.21-1.05 (m, 1H), 0.85-0.56 (m, 2H), M/S: (m/z = 741.3, 2M+Na).

### Example 9

### Preparation of XNW210006

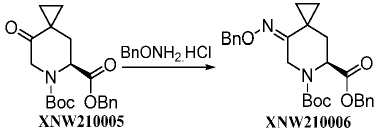

A reaction kettle was purged with nitrogen, and then 370 g of ethanol was added. The temperature was adjusted to 15-25 °C, and 73.7 g (1.5 eq.) of *O*-phenylhydroxylamine hydrochloride was added. 54.2 g (2.0 eq.) of sodium bicarbonate was then added in portions, and the mixture was stirred at this temperature for 1.5 h. 800 g (1.0 eq.) of a solution of XNW210005 in ethanol (prepared according to the method of Example 8) was dropwise added at this temperature, and the mixture was allowed to react for 3 h. Sampling was performed under intermediate control until the reaction was completed.

With the jacket temperature controlled below 60 °C, the mixture was concentrated under reduced pressure until no obvious fraction was observed. 523 g of methyl *tert*-butyl ether and 335.7 g of water were added, and the mixture was stirred at 20-30 °C for 30 min and left to stand for layer separation. 678 g of a 10% aqueous citric acid solution was added to the organic phase, and the mixture was stirred and left to stand for layer separation. 339 g of a 10% aqueous citric acid solution was added to the organic phase, and the mixture was stirred and left to stand for layer separation. Sampling was performed on the organic phase for detection until the reaction was completed. 240 g of a 5% aqueous sodium bicarbonate solution was added to the organic phase, and the mixture was stirred and left to stand for layer separation. With the jacket temperature controlled below 50 °C, the organic phase was concentrated under reduced pressure until no liquid dripped out. 150 g of ethyl acetate was added, and the mixture was further concentrated under reduced pressure until no obvious fraction was observed. Another 290 g of ethyl acetate was then added to give 477.4 g of a solution of XNW210006 in ethyl acetate, with a purity of 78.3%, a content of 28.3%, and a yield of 90%. ¹H NMR, DMSO-*d₆*, 7.37-7.28 (m, 10H), 5.30-5.10 (m, 2H), 5.00-4.69 (m, 1H), 4.26-4.11 (m, 2H), 2.43-2.31 (m, 1H), 2.04-1.98 (m, 1H), 1.59 (s, 2H), 1.49-1.40 (m, 10H), 1.21-1.05 (m, 1H), 0.85-0.56 (m, 2H), M/S: (m/z = 487.2, M+Na).

### Example 10

### Preparation of XNW210007

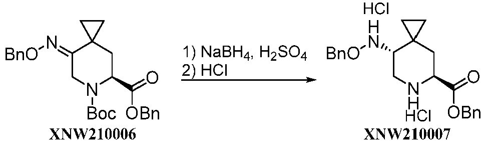

A reaction kettle was purged with nitrogen, and then 684 g of a solution of XNW210006 in ethyl acetate (1 eq., prepared according to the method of Example 6) and 606 g of ethyl acetate were sequentially added. The internal temperature was adjusted to -35 to - 25 °C, and 653 g of a pre-prepared solution of concentrated sulfuric acid in ethyl acetate (383 g (12 eq.) of concentrated sulfuric acid and 270 g of ethyl acetate) was slowly dropwise added at this temperature. The mixture was allowed to react for 21 h, and sampling was performed under intermediate control until the reaction was completed.

The temperature of the system was adjusted to -70 to -60 °C, and 49.03 g (4 eq.) of sodium borohydride was added in portions at this temperature. The mixture was allowed to react for another 4 h, and sampling was performed under intermediate control until the reaction was completed.

The system was slowly heated to 0-10 °C, and with the temperature controlled at 0-10 °C, the reaction solution was slowly dropwise added to 906 g of water which was pre-cooled to 0-10 °C. 667 g of a 25% ammonium hydroxide solution was slowly added with the temperature controlled at 5-25 °C, and the mixture was left to stand for layer separation. With the jacket temperature controlled below 50 °C, the organic phase was concentrated until no obvious fraction was observed. 720 mL of ethanol was added, and the mixture was concentrated until no obvious fraction was observed. Another 720 mL of ethanol was then added, followed by dropwise addition of 215 g of a 4 M solution of hydrogen chloride in ethanol at room temperature. The mixture was allowed to react for 6 h, then stirred at room temperature for crystallization, and filtered. The filter cake was washed with 142 mL of ethanol, and the wet product was dried at 50 °C under vacuum for 12 h to give 90.1 g of XNW210007 as a white solid, with a purity of 92.3%, a content of 97.7%, and a yield of 70%. ¹H NMR, DMSO-*d₆*, 9.90-9.77 (m, 2H), 8.36 (s, 2H), 7.43-7.28 (m, 10H), 5.26 (s, 2H), 4.71-4.67 (m, 2H), 4.34(s, 1H), 3.51-3.45 (m, 1H), 3.11-3.07 (m, 2H), 2.07-2.02 (m, 1H), 1.81-1.76 (m, 1H), 0.70-0.62 (m, 2H), 0.40-0.37 (m, 1H), 0.20-0.17 (m, 1H), M/S: (m/z = 367.2, M-2HCl+H).

### Example 11

### Preparation of XNW210008

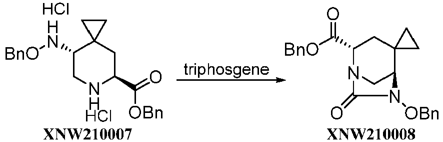

A reaction kettle was purged with nitrogen, and then 135 g of acetonitrile was added and stirred. The internal temperature was adjusted to 10-30 °C, and 15 g of triphosgene was added. The mixture was stirred until triphosgene was completely dissolved, thus giving a 10% solution of triphosgene in acetonitrile for later use.

Another reaction kettle was prepared and purged with nitrogen, and then 156 g of acetonitrile and 51.2 g (1.0 eq.) of XNW210007 were sequentially added. The internal temperature was adjusted to 10-20 °C, and 88.3 g (6.0 eq.) of DIPEA was slowly added with the internal temperature controlled at 10-30 °C. The internal temperature of the system was then adjusted to -10 to 0 °C, and 127.0 g (0.376 eq.) of the above-prepared 10% solution of triphosgene in acetonitrile was slowly added at this temperature. The mixture was allowed to react for 1 h, and sampling was performed under intermediate control until the reaction was completed (if the intermediate control was not satisfactory, additional solution of triphosgene in acetonitrile was added according to the intermediate control result). The temperature of the system was adjusted to 30-40 °C, and the mixture was allowed to react for another 8 h. Sampling was performed under intermediate control until the reaction was completed.

The temperature of the system was adjusted to 15-25 °C, and 250 g of water and 370 g of methyl *tert*-butyl ether were added at this temperature. The mixture was stirred for 30 min and left to stand for layer separation. 400 g of a mixed aqueous solution of 5% sodium carbonate and 5% sodium chloride was added to the organic phase, and the mixture was stirred for 30 min and left to stand for layer separation. 250 g of a sodium chloride solution was added to the organic phase, and the mixture was stirred for 30 min and left to stand for layer separation. The organic phase was concentrated under reduced pressure to 1 V with the temperature controlled to be no higher than 55 °C, and then 185 g of methyl *tert*-butyl ether was added. The mixture was stirred for 30 min and left to stand for layer separation. The organic phase was concentrated under reduced pressure to 1 V with the temperature controlled to be no higher than 55 °C, and then 197 g of acetone was added. With the temperature controlled to be no higher than 55 °C, the organic phase was further concentrated under reduced pressure until no obvious liquid flowed out, thus giving 55.3 g of XNW210008 as an oil, with a purity of 87.6%, a content of 77%, and a yield of 93%.

### Example 12

### Preparation of XNW210009

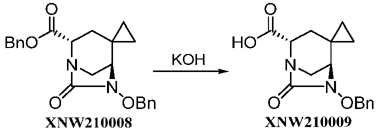

### Scheme 1

A reaction kettle was purged with nitrogen, and then 442 mL of acetone, 55.3 g (1.0 eq.) of XNW210008, and 110 mL of water were sequentially added. The internal temperature was adjusted to -10 to 0 °C, and 171.65 g (0.9 eq.) of an 85% aqueous potassium hydroxide solution was slowly added at this temperature. The mixture was allowed to react for 2 h, and sampling was performed under intermediate control until the reaction was completed.

With the temperature controlled at -10 to 10 °C, 100 mL of water and 150 mL of methyl *tert*-butyl ether were added. The mixture was stirred and left to stand for layer separation. The aqueous phase was washed with 150 mL of methyl *tert*-butyl ether, and then washed twice with isopropyl acetate (150 mL × 2). 15 mL of 31% concentrated hydrochloric acid was added to the aqueous phase to adjust the pH to 1-2, and 250 mL of ethyl acetate was added. The mixture was stirred and left to stand for layer separation. 150 mL of ethyl acetate was further added to the aqueous phase, and the mixture was stirred and left to stand for layer separation. The two ethyl acetate phases were combined and concentrated under reduced pressure at 55 °C or lower to 1-2 V, and then 150 mL of ethyl acetate was added. The organic phase was concentrated under reduced pressure at 55 °C or lower to 1-2 V, and then 200 mL of methyl *tert*-butyl ether was added. The organic phase was concentrated under reduced pressure at 55 °C or lower to 2 V, and then another 200 mL of methyl *tert*-butyl ether was added. The mixture was cooled to 15-25 °C in a gradient manner, then stirred for 2 h, and filtered. The wet product was washed with 25 mL of methyl *tert*-butyl ether, and then dried under vacuum at 50 °C to give 21 g of XNW210009, with a purity of 97.2%, a content of 94.6%, and a yield of 59%. (¹H NMR, DMSO-*d₆*, 12.87(s, 1H), 7.45-7.33 (m, 5H), 4.94-4.86 (m, 2H), 3.98-3.96 (m, 1H), 3.01-2.92 (m, 2H), 2.91(s, 1H), 2.27-2.19 (m, 1H), 1.49-1.44 (m, 1H), 0.57-0.52 (m, 1H), 0.39-0.31 (m, 2H), 0.26-0.23 (m, 1H)), M/S: (m/z = 627.2, 2M+Na).

### Scheme 2

A reaction kettle was purged with nitrogen, and then 150 mL of acetone, 20 g (1.0 eq.) of XNW210008, and 40 mL of water were sequentially added. The internal temperature was adjusted to -10 to 0 °C, and 63.1 g (1.0 eq.) of an 85% aqueous potassium hydroxide solution was slowly added at this temperature. The mixture was allowed to react for 2 h, and sampling was performed under intermediate control until the reaction was completed.

With the temperature controlled at -10 to 10 °C, 40 mL of water was added. The mixture was extracted with 100 mL of methyl *tert*-butyl ether, and the aqueous phase was washed twice with ethyl acetate (60 mL × 3). 100 mL of dichloromethane was added to the aqueous phase, and the mixture was adjusted to pH 1-2 with 31% concentrated hydrochloric acid, followed by layer separation. The aqueous phase was extracted with 60 mL of dichloromethane, and the two dichloromethane phases were combined. The organic phase was washed with a 5% sodium chloride solution (60 mL × 2) and concentrated under reduced pressure to 1-2 V. 100 mL of methyl *tert*-butyl ether was added, and the organic phase was concentrated under reduced pressure to 2 V, then cooled to 15-25 °C, stirred for 20 h, and filtered. The wet product was washed with 10 mL of methyl *tert*-butyl ether, and then dried under vacuum at 50 °C to give 10.63 g of XNW210009 as a white solid, with a purity of 96% and a yield of 69%. (¹H NMR, DMSO-*d₆*, 12.87(s, 1H), 7.45-7.33 (m, 5H), 4.94-4.86 (m, 2H), 3.98-3.96 (m, 1H), 3.01-2.92 (m, 2H), 2.91(s, 1H), 2.27-2.19 (m, 1H), 1.49-1.44 (m, 1H), 0.57-0.52 (m, 1H), 0.39-0.31 (m, 2H), 0.26-0.23 (m, 1H)), M/S: (m/z = 627.2, 2M+Na).

### Example 13

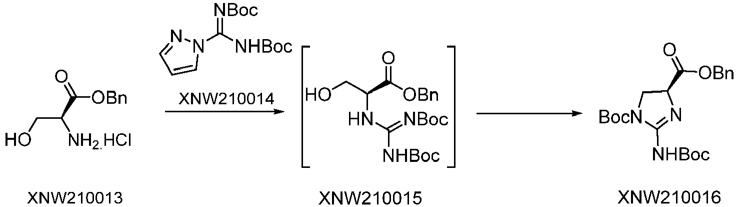

A reaction kettle was purged with nitrogen, and then 266 g of dichloromethane, 20 g (1.0 eq.) of XNW210013, 25.34 g (1.0 eq.) of XNW210014, 10.42 g (1.3 eq.) of potassium bicarbonate, and 20 g of water were sequentially added. The internal temperature was adjusted to 20-30 °C and the mixture was allowed to react at this temperature for 16 h. Sampling was performed under intermediate control until the reaction was completed.

180 g of water was added to the system, and the mixture was stirred for 30 min and left to stand for 30 min for layer separation. 100 g of water was added to the organic phase for washing, and with the temperature controlled below 45 °C, the organic phase was concentrated under reduced pressure to 2-3 V 266 g of dichloromethane was then added to the concentrate to give a solution of XNW210015 in dichloromethane.

532 g of dichloromethane, 27.28 g (1.3 eq.) of triphenylphosphine, and 14.16 g (2.6 eq.) of imidazole were added into another reaction kettle under nitrogen. The internal temperature was adjusted to -5 to 5 °C, and 26.4 g (1.3 eq.) of iodine was added to the system in portions at this temperature. The mixture was stirred for 1 h, and the solution of XNW210015 in dichloromethane was dropwise added to the reaction system at this temperature. The mixture was allowed to react for 1 h, and sampling was performed under intermediate control until the reaction was completed.

210 g of a 5% Na₂SO₃ solution was added to the reaction solution at -5 to 5 °C, and the mixture was naturally heated to room temperature and then left to stand for layer separation. The organic phase was washed with 100 g of water, and with the temperature controlled below 45 °C, the organic phase was concentrated under reduced pressure to 2-3 V 286 g of DMF and 20 g of *n*-heptane were then added to the concentrate, and the mixture was further concentrated until no solvent was evaporated. The temperature of the concentrate was adjusted to 20-30 °C, and 300 g of water was dropwise added at this temperature. The mixture was stirred at this temperature for 2 h or longer and then filtered. The filter cake was washed with 300 g of DMF/water (1:1). The wet product and 364 g of ethyl acetate were added into a reaction kettle, and the mixture was stirred for 2 h or longer at 20-30 °C until the wet product was completely dissolved. 200 g of water was added at this temperature, and the mixture was left to stand for layer separation. The organic phase was washed with 100 g of water, and with the temperature controlled below 50 °C, the organic phase was concentrated under reduced pressure to 2-3 V 55 g of *n*-heptane and 59 g of methyl *tert*-butyl ether were then added to the concentrate, and the mixture was stirred at room temperature for 2 h and filtered. The filtrate was washed with 30 g of methyl *tert*-butyl ether. The wet product was dried under vacuum at 50 °C to give 21.1 g of XNW210016, with a purity of 99.8%, an ee value of greater than 99%, and a yield of 58.3%. ¹H NMR, DMSO-*d₆*, 9.00 (s, 1H), 7.38-7.31 (m, 5H), 5.23-5.13 (m, 2H), 4.48-4.46 (m, 1H), 4.02-3.76 (m, 2H), 1.44-1.41 (m, 18H).

### Example 14

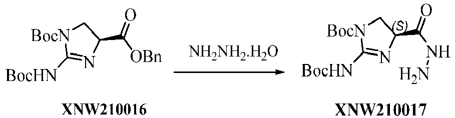

### Scheme 1

A reaction kettle was purged with nitrogen, and then 900 g (1.0 eq.) of XNW210016 and 10670 g of methanol were sequentially added. The mixture was stirred, and the system was cooled to -20 to 10 °C. 405 g (3.0 eq.) of 80% hydrazine hydrate was dropwise added with the temperature controlled to be no higher than 5 °C, and the mixture was allowed to react at -5 to 5 °C for 1 h. 9000 g of a 10% ammonium chloride solution was dropwise added with the temperature controlled to be no higher than 5 °C, and then 11880 g of dichloromethane was added at this temperature. The mixture was stirred and left to stand for layer separation. The organic phase was collected, and the aqueous phase was extracted with 3560 g of dichloromethane. The organic phases were then combined, washed with 9000 g of water, and concentrated under reduced pressure at 40 °C or lower to 2-4 times the volume. 11880 g of dichloromethane was added, and the mixture was further concentrated under reduced pressure to 2-4 times the volume. 5940 g of dichloromethane was then added, thus giving 9720 g of a solution of XNW210017 in dichloromethane, with a purity of 95.9%, a content of 7.3%, and a yield of 96.3%. ¹H NMR, CDCl₃, 9.708 (s, 1H), 8.098 (s, 1H), 4.598-4.643 (m, 1H), 3.974-4.021 (t, 1H), 3.947-3.968 (d, 1H), 3.895 (s, 2H), 1.151-1.522 (m, 18H), M/S: (m/z = 344.1939, M+H).

### Scheme 2

A reaction kettle was purged with nitrogen, and then 900 g (1.0 eq.) of XNW210016, 2133 g of methanol, and 11880 g of dichloromethane were sequentially added. The mixture was stirred, and the system was cooled to -5 to 5 °C. 405 g (3.0 eq.) of 80% hydrazine hydrate was dropwise added at this temperature, and the mixture was allowed to react at -5 to 5 °C for 4-6 h. Sampling was performed under intermediate control until the reaction was completed. 4500 g of a 10% ammonium chloride solution was then dropwise added with the temperature controlled at -5 to 5 °C, and the mixture was stirred and left to stand for layer separation. The organic phase was temporarily stored, and the aqueous phase was extracted with 3564 g of dichloromethane. The two organic phases were then combined, washed with 9000 g of water, and concentrated under reduced pressure at 40 °C or lower to 2-4 times the volume. 11880 g of dichloromethane was added, and the mixture was further concentrated under reduced pressure to 2-4 times the volume. 5940 g of dichloromethane was then added, thus giving 9548 g of a solution of XNW210017 in dichloromethane, with a purity of 96.1%, a content of 7.5%, and a yield of 97.2%. ¹H NMR, CDCl₃, 9.708 (s, 1H), 8.098 (s, 1H), 4.598-4.643 (m, 1H), 3.974-4.021 (t, 1H), 3.947-3.968 (d, 1H), 3.895 (s, 2H), 1.151-1.522 (m, 18H), M/S: (m/z = 344.1939, M+H).

### Example 15

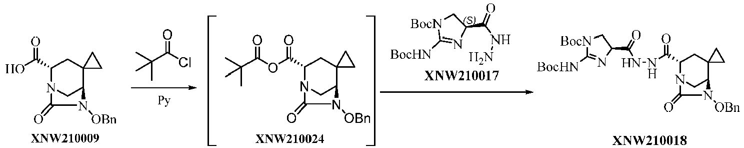

A reaction kettle was purged with nitrogen, and then 500 g (1.0 eq.) of XNW210009 and 6600 g of dichloromethane were sequentially added. The mixture was cooled to -5 °C, and 260 g (2.0 eq.) of pyridine was added, followed by dropwise addition of 220 g (1.1 eq.) of pivaloyl chloride at -5 °C or lower. After the dropwise addition, the mixture was allowed to react at -10 to 0 °C for 2.5 h, and sampling was performed under intermediate control until the reaction was completed, thus giving a solution of XMW210024 in dichloromethane for later use.

Another reaction kettle was purged with nitrogen, and then 9720 g (1.2 eq.) of a solution of XNW210017 in dichloromethane (with a content of 7.3%) was added. The system was cooled to -10 to 0 °C, and then the solution of XNW210024 in dichloromethane obtained above was dropwise added with the temperature controlled at 0 °C or lower. After the dropwise addition, the mixture was allowed to react at -10 to 0 °C for 1 h, and sampling was performed under intermediate control until the reaction was completed. 5000 g of water was dropwise added with the temperature controlled below 10 °C, and the mixture was stirred and left to stand for layer separation. The organic phase was collected, washed with 5000 g of a 5% aqueous sodium bicarbonate solution, and then concentrated under reduced pressure with the temperature controlled below 45 °C to 2-4 times the volume. 4500 g of ethyl acetate was added, and the mixture was further concentrated to 4 times the volume. 9000 g of ethyl acetate and 2500 g of water were then added, and the mixture was stirred and left to stand for layer separation. The organic phase was concentrated under reduced pressure to 4-6 times the volume, and 3700 g of methyl *tert*-butyl ether was added at 35-45 °C. The mixture was stirred at this temperature for 3 h, then cooled to 10-20 °C, stirred for another 3 h, and filtered. The wet product was washed with a mixed solution of 450 g of ethyl acetate and 370 g of methyl *tert*-butyl ether, and then dried to give 845 g of XNW210018, with a purity of 98.8% and a yield of 79.2%. ¹H NMR, CDCl₃, 9.897 (s, 1H), 9.865 (s, 1H), 8.966 (s, 1H), 7.359-7.454 (m, 5H), 4.917 (s, 2H), 3.971-3.998 (d, 1H), 3.944-3.964 (d, 1H), 3.777-3.880 (m, 2H), 3.751-3.761 (s, 1H), 2.990-3.012 (d, 2H), 2.094-2.151 (dd, 1H), 1.551-1.588 (d, 1H), 1.410-1.470 (m, 18H), 0.556-0.580 (d, 1H), 0.339-0.395 (dd, 2H), 0.202-0.235 (d, 1H), M/S: (m/z = 628.3166, M+H).

Preparation and screening of intermediates:
(1) the preparation was carried out as described above, except that triphosgene (0.47 eq.) was used in place of pivaloyl chloride and the reaction temperature was -10 to 5 °C, resulting in a yield of 60% for the corresponding intermediate;
(2) the preparation was carried out as described above, except that *N*,*N*'-disuccinimidyl carbonate (1.06-1.8 eq.) was used in place of pivaloyl chloride and the reaction temperature was -10 to 35 °C, resulting in a yield of 43%-55%.

### Example 16

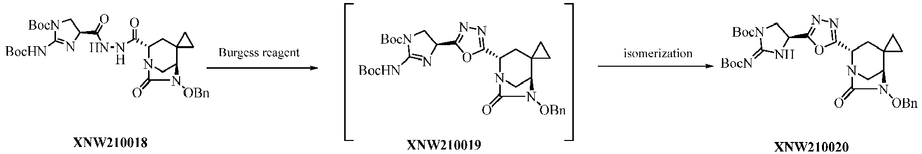

A reaction kettle was purged with nitrogen, and then 1200 g (4.63 eq.) of Burgess reagent, 5670 g of ethyl acetate, 570 g (4.1 eq.) of *N*,*N*-diisopropylethylamine, and 700 g (1.0 eq.) of XNW210018 were sequentially added. The mixture was allowed to react at 25-35 °C for 20 h, and sampling was performed under intermediate control until the reaction was completed. 3500 g of water was then added with the temperature controlled at 15-25 °C, and the mixture was stirred and left to stand for layer separation. The organic phase was collected and washed with 7000 g of a 3% aqueous sodium chloride solution, and then 7000 g of water was added to the organic phase. With the temperature controlled at 15-25 °C, the aqueous phase in the system was adjusted to pH 6-8 with 5% citric acid. The system was then heated to 35-45 °C and allowed to react for 20 h, and sampling was performed under intermediate control until the reaction was completed. The system was cooled to 15-25 °C, and 2390 g of *n*-heptane was dropwise added. The mixture was stirred for 3 h, then cooled to 5-15 °C, stirred for another 3 h, and filtered. The wet product was rinsed with 2800 g of water and then dried to give 510 g of XNW210020 as a white solid, with a purity of 98.74% and a yield of 77.2%. (¹H NMR, CDCl₃, 9.730 (s, 1H), 7.357-7.429 (m, 5H), 5.400-5.433 (m, 1H), 4.891-5.070 (dd, 2H), 4.795-4.814 (d, 1H), 4.074-4.096 (m, 2H), 3.680 (s, 1H), 2.706-2.995 (d, 2H), 2.441-2.686 (dd, 1H), 1.711-1.749 (d, 1H), 1.337-1.537 (m, 18H), 0.780-0.802 (d, 1H), 0.542-0.566 (d, 1H), 0.448-0.471 (d, 1H), 0.155-0.179 (d, 1H)), M/S: (m/z = 610.1979, M+H).

### Example 17

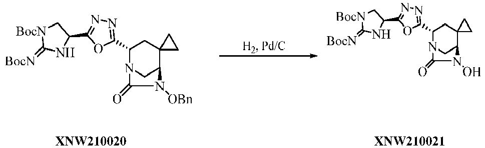

### Scheme 1

A reaction kettle was purged with nitrogen, and then 8880 g of tetrahydrofuran, 400 g (1.0 eq.) of XNW210020, and 36 g (0.09 weight equivalent) of 10% palladium on carbon (dry basis) were sequentially added. With the temperature controlled at 10-20 °C, the mixture was allowed to react under a hydrogen pressure of 0.3-0.5 Mpa for 24 h. Sampling was performed under intermediate control until the reaction was completed. 4400 g of dimethyl sulfoxide was added, and the mixture was heated to 15-30 °C, stirred for 60 min, and filtered. The filter cake was washed with 712 g of tetrahydrofuran, and the filtrates were combined. 32 g of mercaptoalkyl functionalized silica and 32 g of activated carbon were then added, and the mixture was stirred at 15-25 °C for 4 h and filtered. The filter cake was washed with 712 g of tetrahydrofuran, and the filtrates were combined, and then concentrated under reduced pressure to 10-11 times the volume with the temperature controlled below 30 °C. 3200 g of water and 2520 g of acetonitrile were then dropwise added at 10-25 °C, and the mixture was stirred for 3 h and filtered. The wet product was rinsed with 1200 g of water and then dried to give 299 g ofXNW210021, with a purity of 95.7% and a yield of 87.7%. (¹H NMR, CDCl₃, 9.739 (s, 1H), 5.389-5.433 (dd, 1H), 4.790-4.809 (d, 1H), 4.074-4.194 (m, 2H), 3.180-3.218 (m, 1H), 3.096-3.125 (d, 1H), 2.990-2.999 (d, 1H), 2.670-2.728 (dd, 1H), 1.791-1.829 (d, 1H), 1.485-1.541 (m, 18H), 0.778-0.879 (m, 2H), 0.503-0.554 (d, 1H), 0.330-0.367 (m 1H)), M/S: (m/z = 520.1554, M+H).

### Scheme 2

A reaction kettle was purged with nitrogen, and then 3090 g of *N*-methyl-2-pyrrolidone, 200 g (1.0 eq.) of XNW210020, and 18 g (0.09 weight equivalent) of 10% palladium on carbon (dry basis) were sequentially added. With the temperature controlled at 10-20 °C, the mixture was allowed to react under a hydrogen pressure of 0.8-1.2 Mpa for 10-15 h. Sampling was performed under intermediate control until the reaction was completed. The mixture was filtered, and the filter cake was washed with 412 g of *N*-methyl-2-pyrrolidone. The filtrates were combined, and 16 g of mercaptoalkyl functionalized silica and 16 g of activated carbon were added. The mixture was stirred at 15-25 °C for 4 h and filtered. The filter cake was washed with 412 g of *N*-methyl-2-pyrrolidone, and the filtrates were combined. 6000 g of purified water was then dropwise added with the temperature controlled at 10-25 °C, and the mixture was stirred at this temperature for 2-6 h and filtered. The wet product was rinsed with 800 g of water and then dried to give 161.9 g of XNW210021, with a purity of 97.2% and a yield of 95.0%. (¹H NMR, CDCl₃, 9.739 (s, 1H), 5.389-5.433 (dd, 1H), 4.790-4.809 (d, 1H), 4.074-4.194 (m, 2H), 3.180-3.218 (m, 1H), 3.096-3.125 (d, 1H), 2.990-2.999 (d, 1H), 2.670-2.728 (dd, 1H), 1.791-1.829 (d, 1H), 1.485-1.541 (m, 18H), 0.778-0.879 (m, 2H), 0.503-0.554 (d, 1H), 0.330-0.367 (m 1H)), M/S: (m/z = 520.1554, M+H).

### Example 18

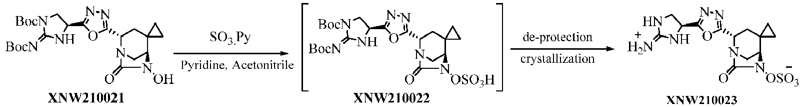

A reaction kettle was purged with nitrogen, and then 20 g (1.0 eq.) of XNW210021, 79 g of acetonitrile, 10.66 g (3.5 eq.) of pyridine, and 21.44 g (3.5 eq.) of sulfur trioxide pyridine were sequentially added. The temperature was adjusted to 25-35 °C, and the mixture was allowed to react for 6 h. Sampling was performed under intermediate control until the reaction was completed. The system was cooled to 15-25 °C, and 1.6 g of activated carbon was added. The mixture was stirred at 15-25 °C for 1 h and filtered, and the filter cake was rinsed with 30 g of acetonitrile. The filtrates were combined, and 200 g of water was added. The mixture was concentrated under reduced pressure to 9-11 times the volume with the temperature controlled below 45 °C, and then 79 g of acetonitrile and 20 g of water were added. The mixture was further concentrated to 9-11 times the volume and allowed to react at 30-40 °C for 5 h. Sampling was performed under intermediate control until the reaction was completed. The reaction solution was cooled to 0-10 °C, stirred for 3 h, and filtered. The filter cake was washed with 40 g of a cold 10% aqueous acetonitrile solution, and the wet product was dried to give 10.8 g of XNW210023 as an off-white solid, with a purity of 98.7% and a yield of 70%. (¹H NMR, DMSO-*d₆*, 8.731(s, 1H), 8.263(s, 1H), 8.063 (s, 2H), 5.434-5.472 (dd, 1H), 4.764-4.783 (d, 1H), 4.015-4.066 (t, 1H), 3.862-3.901 (m, 1H), 3.331-3.380 (d, 1H), 3.030-3.069 (dd, 1H), 2.846-2.876 (d, 1H), 2.401-2.458 (m, 1H), 1.661-1.700 (d, 1H), 0.626-0.635 (d, 1H), 0.579-0.606 (d, 1H), 0.431-0.454 (d, 1H), 0.361-0.419 (d, 1H)), MIS: (m/z = 400.1065, M+H).

Although specific embodiments of the present invention have been described above, it will be appreciated by those skilled in the art that these embodiments are merely illustrative and that many changes or modifications can be made to these embodiments without departing from the principles and spirit of the present invention. The scope of protection of the present invention is therefore defined by the appended claims.

## Claims

1. A preparation method for a β-lactamase inhibitor, comprising the following steps: 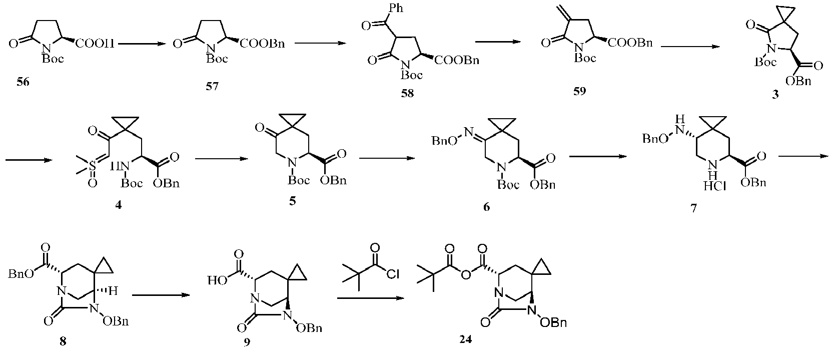 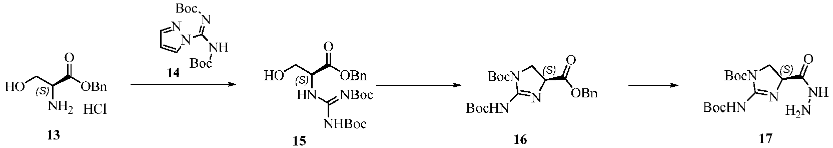 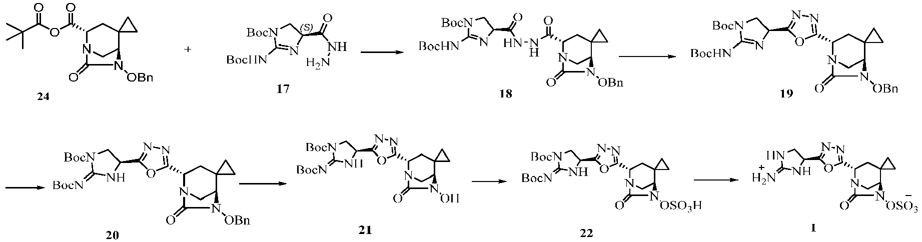
step 1: in a solvent, in the presence of a base and a phase transfer catalyst, subjecting a compound of formula 56 to a benzyl esterification reaction as shown with benzyl bromide to give a compound of formula 57;
step 2: in a solvent, in the presence of a base, subjecting the compound of formula 57 to a benzoylation reaction as shown with benzoyl chloride to give a compound of formula 58;
step 3: in a solvent, in the presence of a base, an aqueous formaldehyde solution or paraformaldehyde, and a phase transfer catalyst, subjecting the compound of formula 58 to a reaction as shown to give a compound of formula 59;
step 4: in a solvent, in the presence of a Zn/Cu or Zn/Ag reagent, subjecting the compound of formula 59 to a cyclopropanation reaction as shown with a methylating reagent to give a compound of formula 3;
step 5: in a solvent, in the presence of a base, subjecting the compound of formula 3 to a ring-opening reaction as shown with trimethylsulfoxonium iodide to give compound 4;
step 6: in a solvent, in the presence of a catalyst and a ligand, subjecting the compound of formula 4 to a ring-closing reaction as shown to give compound 5;
step 7: in a solvent, in the presence of a base, subjecting the compound of formula 5 to a Schiff base reaction with O-phenylhydroxylamine or a salt thereof to give compound 6;
step 8: (1) in a solvent, in the presence of sulfuric acid, reacting the compound of formula 6 with an acid to give a mixture A; and (2) adding a reducing agent to the mixture A to conduct a reduction reaction to give a compound of formula 7;
step 9: adding a mixture of triphosgene and a solvent to a mixture of the compound of formula 7 and/or a salt thereof, a base and a solvent to conduct an amidation ring-closing reaction as shown to give a diazabicyclooctane compound of formula 8;
step 10: in an organic solvent and water, adding a base to the compound of formula 8 for hydrolysis to give a compound of formula 9;
step 11: in a solvent, in the presence of a base, subjecting the carboxylic acid compound of formula 9 to an acylation reaction with pivaloyl chloride to give an anhydride compound of formula 24;
step 12: in an organic solvent and water, in the presence of a base, subjecting a compound of formula 13 to an imidization reaction as shown with a compound of formula 14 to give a guanidine compound of formula 15;
step 13: in a solvent, in the presence of triphenylphosphine, imidazole and iodine, subjecting the guanidine compound of formula 15 to an Appel reaction and a ring-closing reaction as shown to give an azacycle compound of formula 16 and/or a tautomer thereof;
step 14: subjecting a hydrazine to a hydrazidation reaction as shown with the azacycle compound of formula 16 and/or the tautomer thereof to give a hydrazide compound of formula 17 and/or a tautomer thereof;
step 15: subjecting the anhydride compound of formula 24 to an amidation reaction with the hydrazine compound of formula 17 and/or the tautomer thereof to give a hydrazide compound of formula 18 and/or a tautomer thereof;
step 16: in a solvent, in the presence of a dehydrating agent and a base, subjecting the hydrazide compound of formula 18 and/or the tautomer thereof to a ring-closing reaction as shown to give an oxadiazazole compound of formula 19 and/or a tautomer thereof;
step 17: in an organic solvent and water, under an acidic condition, subjecting the hydrazide compound of formula 19 to a tautomerization reaction as shown to give a tautomer thereof, namely an oxadiazazole compound of formula 20;
step 18: in a solvent, in the presence of a palladium catalyst and hydrogen, subjecting the imidazoline compound of formula 20 and/or a tautomer thereof to a debenzylation reaction as shown to give a hydroxylamine compound of formula 21 and/or a tautomer thereof;
step 19: in a solvent, in the presence of pyridine and a sulfonation reagent, subjecting the hydroxylamine compound of formula 21 and/or the tautomer thereof to a sulfonation reaction as shown to give a sulfonic acid oxygen compound of formula 22 and/or a tautomer thereof; and
step 20: in water and an organic solvent, subjecting the sulfonic acid oxygen compound of formula 22 and/or the tautomer thereof to an amine protecting group-removing reaction as shown to give an imine compound of formula I.

2. The preparation method according to claim 1, wherein
in step 1, one or more of the following conditions are met:
(1) the solvent is an ester solvent, such as ethyl acetate;
(2) the base is an alkali metal carbonate, such as potassium carbonate; and
(3) the phase transfer catalyst is tetrabutylammonium bromide;
in step 2, one or more of the following conditions are met:
(1) the solvent is one or more of a ketone solvent, a nitrile solvent, an ether solvent, an ester solvent, and a halogenated hydrocarbon solvent, wherein the ketone solvent can be acetone, the nitrile solvent can be acetonitrile, the ether solvent can be tetrahydrofuran, the ester solvent can be ethyl acetate, and the halogenated hydrocarbon solvent can be dichloromethane;
(2) the volume-to-mass ratio of the solvent to the compound of formula 57 is 5 mL/g to 10 mL/g;
(3) the molar ratio of the benzoyl chloride to the compound of formula 57 is 1.05:1 to 1.1:1;
(4) the base is an alkali metal alkoxide and/or lithium bis(trimethylsilyl)amide, wherein the alkali metal alkoxide can be one or more of sodium *tert*-butoxide, lithium *tert*-butoxide, and potassium *tert*-butoxide;
(5) the molar ratio of the base to the compound of formula 57 is 1.4:1 to 2.5:1;
(6) the benzoylation reaction is conducted under an inert gas, such as nitrogen or argon;
(7) the benzoylation reaction is conducted at a temperature of -65 °C to 25 °C, such as - 15 °C to -5 °C; and
(8) the method for preparing the compound of formula 58 comprises the following step: at -15 °C to 5 °C, sequentially adding the base and the benzoyl chloride to a mixture of the compound of formula 57 and the solvent to conduct the benzoylation reaction to give the compound of formula 58;
in step 3, one or more of the following conditions are met:
(1) the solvent is one or more of an ether solvent, a halogenated hydrocarbon solvent, a nitrile solvent, a ketone solvent, an ester solvent, and *N,N-*dimethylformamide, wherein the ether solvent can be tetrahydrofuran and/or 1,4-dioxane, the halogenated hydrocarbon solvent can be dichloromethane, the ester solvent can be ethyl acetate, the nitrile solvent can be acetonitrile, and the ketone solvent can be acetone;
(2) the volume-to-mass ratio of the solvent to the compound of formula 58 is 8 mL/g to 12 mL/g;
(3) the phase transfer catalyst is a quaternary ammonium salt, such as tetrabutylammonium iodide and/or tetrabutylammonium fluoride trihydrate;
(4) the molar ratio of the phase transfer catalyst to the compound of formula 58 is 0.04:1 to 0.05:1, such as 0.045:1;
(5) the base is an inorganic base and/or an organic base, wherein the inorganic base can be one or more of an alkali metal carbonate, an alkali metal bicarbonate, and an alkali metal alkoxide, wherein the alkali metal carbonate can be potassium carbonate and/or cesium carbonate, the alkali metal bicarbonate can be potassium bicarbonate and/or sodium bicarbonate, and the alkali metal alkoxide can be potassium *tert*-butoxide; the organic base can be triethylamine and/or diisopropylamine;
(6) the molar ratio of the base to the compound of formula 58 is 2:1 to 3:1;
(7) the molar ratio of formaldehyde in the aqueous formaldehyde solution or the paraformaldehyde to the compound of formula 58 is 1:1 to 6:1, such as 3.4:1;
(8) in the step, the base and the aqueous formaldehyde solution or the paraformaldehyde are added in portions, for example, in 3 equal portions;
(9) the reaction is conducted under an inert gas, such as nitrogen or argon; and
(10) the reaction is conducted at a temperature of 15 °C to 55 °C, such as 35 °C to 45 °C;
in step 4, one or more of the following conditions are met:
(1) the solvent is an amide solvent, such as DMF;
(2) the volume-to-mass ratio of the solvent to the compound of formula 59 is 5 mL/g to 30 mL/g, such as 10 mL/g;
(3) the molar ratio of the Zn/Cu or Zn/Ag reagent to the compound of formula 59 is 0.5:1 to 3:1, such as 2:1;
(4) the methylating reagent is dibromomethane and/or diiodomethane;
(5) the molar ratio of the methylating reagent to the compound of formula 59 is 1.5:1 to 3:1, such as 2:1;
(6) the cyclopropanation reaction is conducted under an inert gas, such as nitrogen or argon; and
(7) the cyclopropanation reaction is conducted at a temperature of 15 °C to 45 °C, such as 30 °C to 40 °C;
in step 5, one or more of the following conditions are met:
(1) the solvent is a mixture of an ether solvent and dimethyl sulfoxide, wherein the ether solvent can be tetrahydrofuran;
(2) the volume ratio of the ether solvent to the dimethyl sulfoxide is 1:1.2 to 3:1, such as 5:3;
(3) the base is an alkali metal alkoxide, such as potassium *tert*-butoxide;
(4) the molar ratio of the base to the compound of formula 3 is 1:1 to 1.4:1, such as 1.15:1 to 1.2:1;
(5) the molar ratio of the trimethylsulfoxonium iodide to the compound of formula 3 is 1:1 to 1.4:1, such as 1.15:1 to 1.2:1;
(6) the ring-opening reaction is conducted under an inert gas, wherein the inert gas can be argon or nitrogen;
(7) the ring-opening reaction is conducted at a temperature of -20 to 30 °C; and
(8) the step comprises: mixing the base and the trimethylsulfoxonium iodide in a portion of an ether solvent and dimethyl sulfoxide, and then sequentially adding the remaining portion of the ether solvent and the compound of formula 3 to conduct the ring-opening reaction, wherein the mixing can be conducted at a temperature of 20-30 °C, and the ring-opening reaction can be conducted at a temperature of -10 to 0 °C;
in step 6, one or more of the following conditions are met:
(1) the solvent is one or more of an aromatic hydrocarbon solvent, an alcohol solvent, an ether solvent, an amide solvent, and an ester solvent, wherein the aromatic hydrocarbon solvent can be toluene, the alcohol solvent can be *tert*-amyl alcohol and/or *tert*-pentanol, the ether solvent can be cyclopentyl methyl ether and/or tetrahydrofuran, the amide solvent can be DMAc and/or DMF, and the ester solvent can be isopropyl acetate;
(2) the volume-to-mass ratio of the solvent to the compound of formula 4 is 2 mL/g to 3 mL/g;
(3) the catalyst is one or more of chloro(cyclopentadienyl)bis(triphenylphosphine)ruthenium(II), chloro(1,5-cyclooctadiene)iridium(I) dimer, (1,5-cyclooctadiene)(methoxy)iridium(I) dimer, copper acetate, copper(I) iodide, palladium(II) acetate, *trans*-dichlorobis(acetonitrile)palladium(II), and palladium(II) trifluoroacetate, for example, chloro(cyclopentadienyl)bis(triphenylphosphine)ruthenium(II);
(4) the molar ratio of the catalyst to the compound of formula 4 is 0.01 to 0.1, such as 0.015:1;
(5) a ligand is also added in the ring-closing reaction, wherein the ligand can be triphenylphosphine;
(6) a ligand is also added in the ring-closing reaction, wherein the molar ratio of the ligand to the compound of formula 4 is 0.01 to 0.1, such as 0.04:1;
(7) the ring-closing reaction is conducted under an inert gas, such as nitrogen or argon; and
(8) the ring-closing reaction is conducted at a temperature of 60 °C to 110 °C, such as 95 °C to 105 °C;
in step 7, one or more of the following conditions are met:
(1) the solvent is an alcohol solvent and/or an ester solvent, wherein the alcohol solvent can be one or more of ethanol, isopropanol, *tert*-butanol, and *tert*-amyl alcohol, and the ester solvent can be ethyl acetate;
(2) the volume-to-mass ratio of the solvent to the compound of formula 5 is 8 mL/g to 12 mL/g;
(3) the molar ratio of the compound of formula 5 to the *O*-phenylhydroxylamine or the salt thereof is 1.15:1 to 1.5:1, such as 1.5:1;
(4) the salt of the *O*-phenylhydroxylamine is a hydrochloride of the *O-*phenylhydroxylamine;
(5) the base is an alkali metal bicarbonate, such as sodium bicarbonate;
(6) the molar ratio of the base to the salt of the *O*-phenylhydroxylamine is 2:1.5;
(7) the molar ratio of the base to the compound of formula 5 is 1.5:1 to 2:1, such as 2:1;
(8) the Schiff base reaction is conducted under an inert gas, such as nitrogen or argon;
(9) the Schiff base reaction is conducted at a temperature of 15 °C to 45 °C, such as 15 °C to 25 °C; and
(10) the method for preparing compound 6 comprises the following steps: in a solvent, at 15 °C to 25 °C, adding the base to a mixture of the *O*-phenylhydroxylamine or the salt thereof and a solvent, and then adding a mixture of the compound of formula 5 and a solvent to conduct the Schiff base reaction to give compound 6;
in step 8, one or more of the following conditions are met:
(1) in step (1), the solvent is an ester solvent, such as ethyl acetate;
(2) in step (1), the volume-to-mass ratio of the solvent to the compound of formula 6 is 9 mL/g to 15 mL/g, such as 12 mL/g;
(3) in step (1), the molar ratio of the sulfuric acid to compound 6 is 4:1 to 12:1;
(4) in step (1), the sulfuric acid is added in the form of a solution of concentrated sulfuric acid in the solvent, wherein in the solution, the mass ratio of the concentrated sulfuric acid to the solvent can be 1.4:1;
(5) the reaction in step (1) is conducted at a temperature of -45 °C to -5 °C, such as -35 °C to -25 °C;
(6) after the reaction in step (1) is completed, the reduction reaction in step (2) is directly conducted without a post-treatment to prepare compound 7;
(7) in step (2), the reducing agent is sodium borohydride, borane-methyl sulfide complex, and lithium triethylborohydride, for example, sodium borohydride;
(8) in step (2), the molar ratio of the reducing agent to the compound of formula 6 is 4:1 to 6:1, such as 4:1; and
(9) in step (2), the reduction reaction is conducted at a temperature of -70 °C to -35 °C, such as -70 °C to -60 °C;
in step 9, one or more of the following conditions are met:
(1) the solvent is one or more of acetonitrile, a halogenated hydrocarbon solvent, and an aromatic hydrocarbon solvent, wherein the halogenated hydrocarbon solvent is, for example, dichloromethane, and the aromatic hydrocarbon solvent is, for example, toluene;
(2) the mass percentage of triphosgene in the mixture of the triphosgene and the solvent is 10%;
(3) in the mixture of the compound of formula 7 and/or the salt thereof, the base and the solvent, the volume-to-mass ratio of the solvent to the compound of formula 7 and/or the salt thereof is 3 mL/g;
(4) the salt of the compound of formula 7 is a hydrochloride, for example, 2 equivalents of hydrochloric acid;
(5) the base is an organic base, such as *N,N*-diisopropylethylamine and/or triethylamine;
(6) the molar ratio of the base to the compound of formula 7 is 4:1; or the molar ratio of the base to the salt of the compound of formula 7 is 6:1;
(7) the molar ratio of the triphosgene to the compound of formula 7 and/or the salt thereof is 0.35:1 to 0.5:1, such as 0.38:1 to 0.4:1;
(8) the amidation ring-closing reaction is conducted at a temperature of -10 °C to 0 °C;
(9) the amidation ring-closing reaction is conducted under an inert gas, such as nitrogen or argon; and
(10) the preparation method comprises the following steps: adding the base to the mixture of the compound of formula 7 and/or the salt thereof and the solvent at an internal temperature of 10 °C to 30 °C, and then adding a solution of 10% triphosgene in the solvent at -10 to 0 °C to conduct the amidation ring-closing reaction as shown to give the diazabicyclooctane compound of formula 8;
in step 10, one or more of the following conditions are met:
(1) the molar ratio of the diazabicyclooctane compound of formula 8 to the base is 1:1 to 1:0.9;
(2) the organic solvent is acetone;
(3) the volume ratio of the organic solvent to water is 4:1 to 3:1;
(4) the base is an aqueous potassium hydroxide solution, such as an 85% aqueous potassium hydroxide solution;
(5) the hydrolysis reaction is conducted under an inert gas, such as nitrogen or argon; and
(6) the hydrolysis reaction is conducted at a temperature of -10 to 10 °C, such as -10 to 0 °C;
in step 11, one or more of the following conditions are met:
(1) the molar ratio of the carboxylic acid compound of formula 9 to pivaloyl chloride is 1:1 to 1:1.6, such as 1:1.1;
(2) the solvent is one or more of an aromatic hydrocarbon solvent, an ether solvent, an ester solvent, and a halogenated hydrocarbon solvent, wherein the aromatic hydrocarbon solvent can be toluene, the ether solvent can be tetrahydrofuran, the ester solvent can be ethyl acetate, and the halogenated hydrocarbon solvent can be dichloromethane;
(3) the volume-to-mass ratio of the solvent to the compound of formula 9 is 10 mL/g to 20 mL/g;
(4) the base is an organic base, such as one or more of *N,N-*diisopropylethylamine, pyridine, and triethylamine;
(5) the molar ratio of the base to the carboxylic acid compound of formula 9 is 3:1 to 1:1, such as 2:1;
(6) the acylation reaction is conducted under an inert gas, such as nitrogen or argon;
(7) the acylation reaction is conducted at a temperature of -10 to 10 °C, such as -10 to 0 °C;
(8) the base and pivaloyl chloride are sequentially added to a mixture of the compound of formula 9 and the solvent at -5 °C or lower, and then the acylation reaction is conducted at -10 to 0 °C to give the anhydride compound of formula 24; and
(9) after the acylation reaction is completed, the amidation reaction is directly conducted without a post-treatment;
in step 12, one or more of the following conditions are met:
(1) the molar ratio of the compound of formula 13 to the compound of formula 14 is 1:1;
(2) the organic solvent is a nitrile solvent and/or a halogenated hydrocarbon solvent, wherein the nitrile solvent can be acetonitrile, and the halogenated hydrocarbon solvent can be dichloromethane;
(3) the volume-to-mass ratio of the organic solvent to the compound of formula 14 is 6 mL/g to 15 mL/g, such as 8 mL/g to 10 mL/g;
(4) the imidization reaction is conducted under an inert gas, such as nitrogen or argon;
(5) the imidization reaction is conducted at a temperature of 10 °C to 40 °C, such as 20 °C to 30 °C;
(6) the base is an alkali metal carbonate and/or an alkali metal bicarbonate, such as potassium carbonate and/or potassium bicarbonate; and
(7) the mass ratio of the water to the base is (1.5-2.5):1, such as 2:1;
in step 13, one or more of the following conditions are met:
(1) the molar ratio of the triphenylphosphine to the guanidine compound of formula 15 is 1.1:1 to 1.3:1;
(2) the molar ratio of the imidazole to the guanidine compound of formula 15 is 2.2:1 to 2.6:1;
(3) the molar ratio of the iodine to the guanidine compound of formula 15 is 1.1:1 to 1.3:1;
(4) the solvent is a nitrile solvent and/or a halogenated hydrocarbon solvent, wherein the nitrile solvent can be acetonitrile, and the halogenated hydrocarbon solvent can be dichloromethane;
(5) the volume-to-mass ratio of the solvent to the compound of formula 15 is 16 mL/g to 40 mL/g;
(6) the Appel reaction and the ring-closing reaction are conducted at a temperature of - 10 °C to 10 °C, such as -5 °C to 5 °C;
(7) the Appel reaction and the ring-closing reaction are conducted under an inert gas, such as nitrogen or argon;
(8) a mixture of the guanidine compound of formula 15 and the solvent is dropwise added to a mixture of the iodine, the triphenylphosphine, the imidazole and the solvent; and
(9) at -5 °C to 5 °C, the iodine is added to a mixture of the triphenylphosphine, the imidazole and the solvent in portions to give a reaction system 1, and then a mixture of the guanidine compound of formula 15 and the solvent is added to the above reaction system 1 to conduct the Appel reaction and the ring-closing reaction to give the azacycle compound of formula 16 and/or the tautomer thereof;
in step 14, one or more of the following conditions are met:
(1) the molar ratio of the hydrazine to the azacycle compound of formula 16 and/or the tautomer thereof is 1.5:1 to 3:1;
(2) the hydrazine is hydrazine hydrate, such as 80% hydrazine hydrate;
(3) the solvent is one or more of an alcohol solvent, an aromatic hydrocarbon solvent, and a halogenated alkane, wherein the alcohol solvent can be one or more of methanol, ethanol, and isopropanol, the aromatic hydrocarbon solvent can be toluene, and the halogenated alkane can be dichloromethane;
(4) the volume-to-mass ratio of the solvent to the compound of formula 16 is 10 mL/g to 20 mL/g, such as 12 mL/g;
(5) the hydrazidation reaction is conducted under an inert gas, such as nitrogen or argon;
(6) the hydrazidation reaction is conducted at a temperature of -20 to 10 °C, such as -10 to 5 °C; and
(7) with the temperature controlled at 5 °C or lower, the hydrazine is added to a mixture of the azacycle compound of formula 16 and the solvent to conduct the hydrazidation reaction to give the hydrazide compound of formula 17 and/or the tautomer thereof;
in step 15, one or more of the following conditions are met:
(1) the solvent is one or more of an aromatic hydrocarbon solvent, an ether solvent, an ester solvent, and a halogenated hydrocarbon solvent, wherein the aromatic hydrocarbon solvent can be toluene, the ether solvent can be tetrahydrofuran, the ester solvent can be ethyl acetate, and the halogenated hydrocarbon solvent can be dichloromethane;
(2) the volume-to-mass ratio of the solvent to the compound of formula 17 is 10 mL/g to 20 mL/g;
(3) the molar ratio of the anhydride compound of formula 24 to the hydrazine compound of formula 17 and/or the tautomer thereof is 1.3:1 to 1:1.3, such as 1:1.2;
(4) the amidation reaction is conducted at a temperature of -15 °C to 0 °C, such as -10 °C to 0 °C;
(5) the step comprises: with the temperature controlled at -10 to 0 °C, adding a mixture of the anhydride compound of formula 24 and the solvent to a mixture of the compound of formula 17 and the solvent to conduct the amidation reaction to give the hydrazide compound of formula 18 and/or the tautomer thereof;
(6) the amidation reaction is conducted under an inert gas, such as nitrogen or argon; and
(7) the starting materials for the amidation reaction are the anhydride compound of formula 24, the hydrazine compound of formula 17 and/or the tautomer thereof, and the solvent;
in step 16, one or more of the following conditions are met:
(1) the solvent is one or more of an aromatic hydrocarbon solvent, an ether solvent, an ester solvent, and a halogenated hydrocarbon solvent, wherein the aromatic hydrocarbon solvent can be toluene, the ether solvent can be tetrahydrofuran, the ester solvent can be ethyl acetate, and the halogenated hydrocarbon solvent can be dichloromethane;
(2) the volume-to-mass ratio of the solvent to the compound of formula 18 is 6.5 mL/g to 10 mL/g;
(3) the dehydrating agent is Burgess reagent;
(4) the molar ratio of the dehydrating agent to the compound of formula 18 is 2:1 to 10:1, such as 4.6:1;
(5) the base is an organic base, such as *N,N*-diisopropylethylamine and/or triethylamine;
(6) the molar ratio of the base to the compound of formula 18 is 4:1 to 6.5:1, such as 4.1:1 or 6.2:1;
(7) the ring-closing reaction is conducted under an inert gas, such as nitrogen or argon; and
(8) the ring-closing reaction is conducted at a temperature of 10 °C to 45 °C, such as 25 °C to 35 °C;
in step 17, one or more of the following conditions are met:
(1) the organic solvent is one or more of an aromatic hydrocarbon solvent, an ether solvent, an ester solvent, and a halogenated hydrocarbon solvent, wherein the aromatic hydrocarbon solvent can be toluene, the ether solvent can be tetrahydrofuran, the ester solvent can be ethyl acetate, and the halogenated hydrocarbon solvent can be dichloromethane;
(2) the volume ratio of the water to the organic solvent is 1.3:1 to 1:1;
(3) the mass ratio of the water to the compound of formula 19 is 5:1 to 10:1;
(4) the acidic condition is pH 6-8; and
(5) the acidic condition is achieved by adding 5% citric acid for adjustment, wherein the 5% citric acid can be added at a temperature of 15-25 °C;
in step 18, one or more of the following conditions are met:
(1) the solvent is one or more of an alcohol solvent, an ether solvent, an amide solvent, and a halogenated hydrocarbon solvent, wherein the alcohol solvent can be one or more of methanol, ethanol, and isopropanol, the halogenated hydrocarbon solvent can be dichloromethane, the ether solvent can be tetrahydrofuran, and the amide solvent can be dimethylacetamide and/or N-methyl-2-pyrrolidone;
(2) the volume-to-mass ratio of the solvent to the imidazoline compound of formula 20 and/or the tautomer thereof is 15 mL/g to 27 mL/g;
(3) the palladium catalyst is palladium on carbon or palladium hydroxide, such as 10% palladium on carbon;
(4) the mass ratio of the palladium catalyst to the imidazoline compound of formula 20 and/or the tautomer thereof is 0.02:1 to 0.9:1, such as 0.09:1;
(5) the pressure of the hydrogen is 0.3 Mpa to 0.5 Mpa; and
(6) the debenzylation reaction is conducted at a temperature of 0 °C to 40 °C, such as 10 °C to 20 °C;
in step 19, one or more of the following conditions are met:
(1) the solvent is a halogenated hydrocarbon solvent and/or a nitrile solvent, wherein the halogenated hydrocarbon solvent can be dichloromethane, and the nitrile solvent can be acetonitrile;
(2) the volume-to-mass ratio of the solvent to the hydroxylamine compound of formula 21 and/or the tautomer thereof is 5 mL/g to 20 mL/g;
(3) the sulfonation reagent is one or more of sulfur trioxide pyridine, sulfur trioxide triethylamine, and sulfur trioxide trimethylamine;
(4) the mass ratio of the sulfur trioxide pyridine to the hydroxylamine compound of formula 21 and/or the tautomer thereof is 1.2:1 to 6:1;
(5) the mass ratio of the pyridine to the hydroxylamine compound of formula 21 and/or the tautomer thereof is 2.5:1 to 6.25:1; and
(6) the sulfonation reaction is conducted at a temperature of 15 °C to 35 °C, such as 25 °C to 35 °C;
in step 20, one or more of the following conditions are met:
(1) the organic solvent is a nitrile solvent, wherein the nitrile solvent can be acetonitrile;
(2) the volume-to-mass ratio of the organic solvent to the sulfonic acid oxygen compound of formula 22 and/or the tautomer thereof is 9 mL/g to 11 mL/g; and
(3) the amine protecting group-removing reaction is conducted at a temperature of 18 °C to 40 °C, such as 30 °C to 40 °C.

3. A preparation method for a diazabicyclooctane compound of formula 8, comprising the following step:
adding a mixture of triphosgene and a solvent to a mixture of a compound of formula 7 and/or a salt thereof, a base and a solvent to conduct an amidation ring-closing reaction as shown below to give the diazabicyclooctane compound of formula 8: 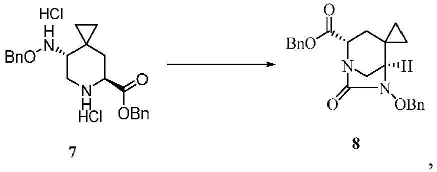
wherein the operations and conditions in the preparation method can be the same as those in the method for preparing the compound of formula 8 in the preparation method according to any one of claims 1 and 2.

4. A preparation method for a compound of formula 7, comprising the following steps:
(1) in a solvent, in the presence of sulfuric acid, reacting a compound of formula 6 with an acid to give a mixture A; and
(2) adding a reducing agent to the mixture A to conduct a reduction reaction to give the compound of formula 7: 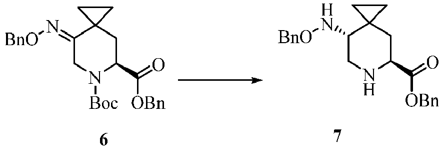
wherein the operations and conditions in the preparation method can be the same as those in the method for preparing the compound of formula 7 in the preparation method according to any one of claims 1 to 2.

5. A preparation method for a compound of formula 6, comprising the following step:
in a solvent, in the presence of a base, subjecting a compound of formula 5 to a Schiff base reaction with O-phenylhydroxylamine or a salt thereof to give the compound of formula 6: 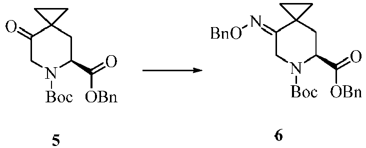
wherein the operations and conditions in the preparation method can be the same as those in the method for preparing the compound of formula 6 in the preparation method according to any one of claims 1 to 2.

6. A preparation method for a compound of formula 5, comprising the following step:
in a solvent, in the presence of a catalyst and a ligand, subjecting a compound of formula 4 to a ring-closing reaction as shown below to give the compound of formula 5: 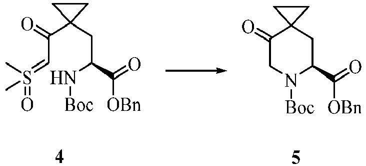
wherein the operations and conditions in the preparation method can be the same as those in the method for preparing the compound of formula 5 in the preparation method according to any one of claims 1 to 2.

7. A preparation method for a compound of formula 3, comprising the following step:
in a solvent, in the presence of a Zn/Cu or Zn/Ag reagent, subjecting a compound of formula 59 to a cyclopropanation reaction as shown below with a methylating reagent to give the compound of formula 3: 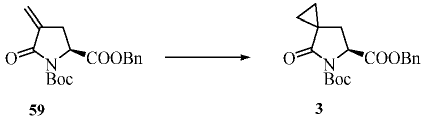
wherein the operations and conditions in the preparation method can be the same as those in the method for preparing the compound of formula 3 in the preparation method according to any one of claims 1 to 2.

8. A preparation method for a compound of formula 59, comprising the following step:
in a solvent, in the presence of a base, an aqueous formaldehyde solution or paraformaldehyde, and a phase transfer catalyst, subjecting a compound of formula 58 to a reaction as shown below to give the compound of formula 59: 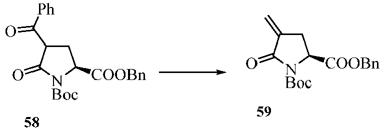
wherein the operations and conditions in the preparation method can be the same as those in the method for preparing the compound of formula 59 in the preparation method according to any one of claims 1 to 2.

9. A preparation method for a compound of formula 58, comprising the following step:
in a solvent, in the presence of a base, subjecting a compound of formula 57 to a benzoylation reaction as shown below with benzoyl chloride to give the compound of formula 58: 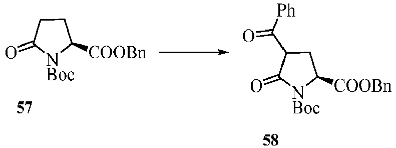
wherein the operations and conditions in the preparation method can be the same as those in the method for preparing the compound of formula 58 in the preparation method according to any one of claims 1 to 2.

10. A preparation method for an oxadiazazole compound and/or a tautomer thereof, comprising the following step (a) and/or step (b):
step (a):
in a solvent, in the presence of a dehydrating agent and a base, subjecting a hydrazide compound of formula 18 and/or a tautomer thereof to a ring-closing reaction as shown to give an oxadiazazole compound of formula 19 and/or a tautomer thereof; 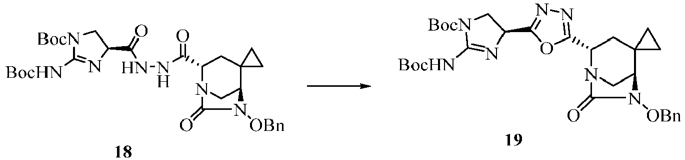
step (b): in an organic solvent and water, under an acidic condition, subjecting a hydrazide compound of formula 19 to a tautomerization reaction as shown to give a tautomer thereof, namely an oxadiazazole compound of formula 20: 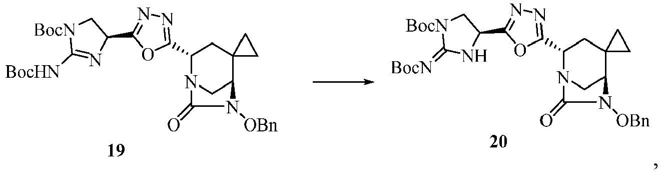
wherein the operations and conditions in the preparation method can be the same as those in the methods for preparing the compounds of formulas 19 and 29 in the preparation method according to any one of claims 1 to 2.

11. A compound of formula 5, 6 or 58, an azacycle compound of formula 16 and/or a tautomer thereof, a hydrazine compound of formula 17 and/or a tautomer thereof, an anhydride compound of formula 24, or a hydrazide compound of formula 18 and/or a tautomer thereof: 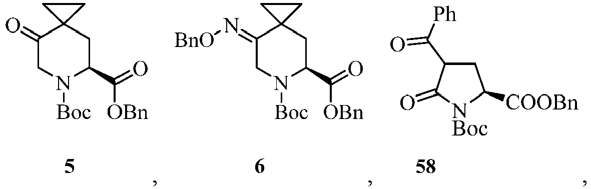 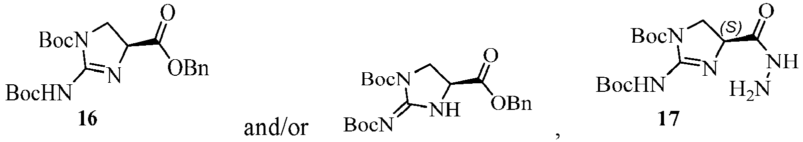 and/or 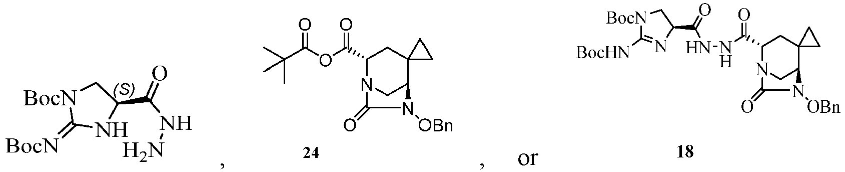 and/or

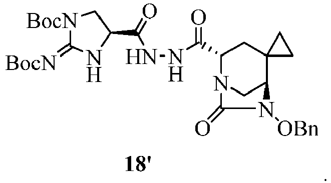

12. A preparation method for a hydrazide compound of formula 18 and/or a tautomer thereof, comprising the following step:
in a solvent, subjecting an anhydride compound of formula 24 to an amidation reaction with a hydrazine compound of formula 17 and/or a tautomer thereof to give the hydrazide compound of formula 18 and/or the tautomer thereof: 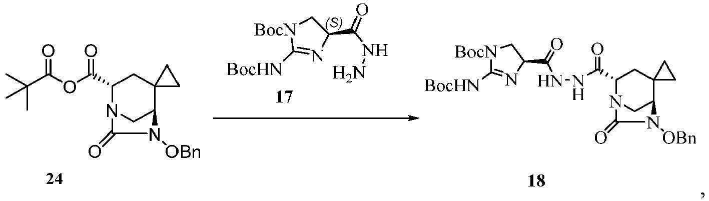
wherein the operations and reaction conditions in the preparation method for the hydrazide compound of formula 18 and/or the tautomer thereof can be the same as those in the method for preparing the hydrazide compound of formula 18 and/or the tautomer thereof in the preparation method according to claim 1 or 2.

13. A preparation method for an anhydride compound of formula 24, comprising the following step:
in a solvent, in the presence of a base, subjecting a carboxylic acid compound of formula 9 to an acylation reaction with pivaloyl chloride to give the anhydride compound of formula 24: 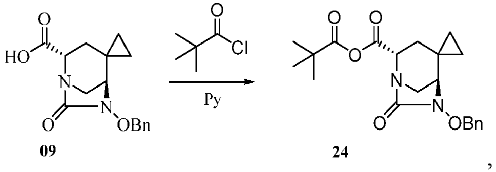
wherein the operations and reaction conditions in the preparation method for the anhydride compound of formula 24 and/or the tautomer thereof can be the same as those in the method for preparing the anhydride compound of formula 24 and/or the tautomer thereof in the preparation method according to any one of claims 1 and 2.

14. A preparation method for a hydrazine compound of formula 17 and/or a tautomer thereof, comprising the following step:
subjecting a hydrazine to a hydrazidation reaction as shown below with an azacycle compound of formula 16 and/or a tautomer thereof to give the hydrazide compound of formula 17 and/or the tautomer thereof: 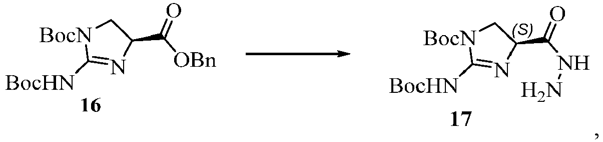
wherein the operations and reaction conditions in the preparation method for the hydrazine compound of formula 17 and/or the tautomer thereof can be the same as those in the method for preparing the hydrazide compound of formula 17 and/or the tautomer thereof in the preparation method according to any one of claims 1 and 2.

15. A preparation method for an azacycle compound of formula 16 and/or a tautomer thereof, comprising the following step:
in a solvent, in the presence of triphenylphosphine, imidazole and iodine, subjecting a guanidine compound of formula 15 to an Appel reaction and a ring-closing reaction as shown below to give the azacycle compound of formula 16 and/or the tautomer thereof: 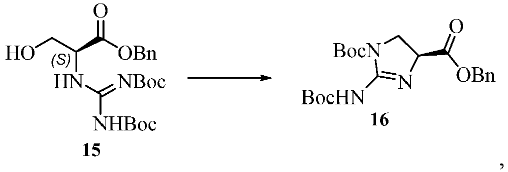
wherein the reaction conditions and operations in the preparation method for the azacycle compound of formula 16 and/or the tautomer thereof can be the same as those in the method for preparing the azacycle compound of formula 16 and/or the tautomer thereof in the preparation method according to any one of claims 1 and 2.

16. A preparation method for a β-lactamase inhibitor and an intermediate, being any one of the following schemes:
scheme 1, a preparation method for a hydroxylamine compound of formula 21 and/or a tautomer thereof, which comprises the following steps:
step (1), comprising the following step (a) and/or step (b):
step (a):
in a solvent, in the presence of a dehydrating agent and a base, subjecting a hydrazide compound of formula 18 and/or a tautomer thereof to a ring-closing reaction as shown to give an oxadiazazole compound of formula 19 and/or a tautomer thereof; 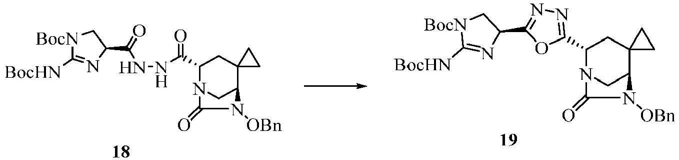
step (b): in a solvent and water, under an acidic condition, subjecting a hydrazide compound of formula 19 to a tautomerization reaction as shown to give a tautomer thereof, namely an oxadiazazole compound of formula 20: 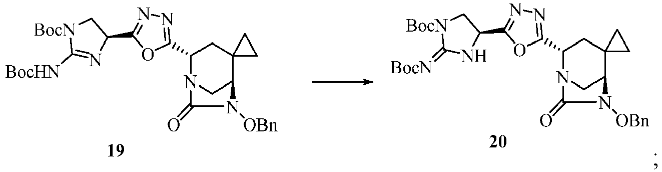 and
step (2): in a solvent, in the presence of a palladium catalyst and hydrogen, subjecting the imidazoline compound of formula 20 and/or a tautomer thereof to a debenzylation reaction as shown to give the hydroxylamine compound of formula 21 and/or the tautomer thereof: 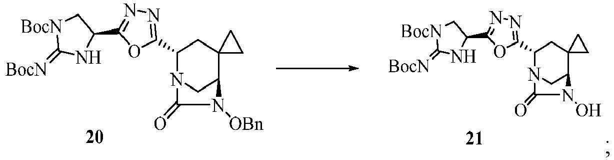
scheme 2, a preparation method for a hydroxylamine compound of formula 21 and/or a tautomer thereof, which comprises the following steps:
step (1): the same as step (1) in scheme 1;
step (2): the same as step (2) in scheme 1; and
step (3): in a solvent, in the presence of pyridine and a sulfonation reagent, subjecting the hydroxylamine compound of formula 21 and/or the tautomer thereof to a sulfonation reaction as shown to give a sulfonic acid oxygen compound of formula 22 and/or a tautomer thereof: 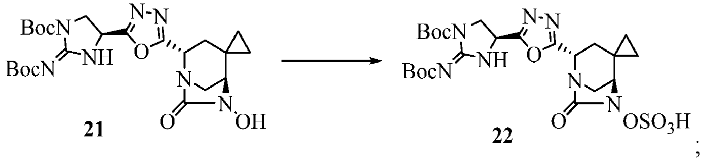 and
scheme 3, a preparation method for an imine compound of formula I, which comprises the following steps:
steps (1) to (3): the same as steps (1) to (3) in scheme 2; and
step (4): in water and an organic solvent, subjecting the sulfonic acid oxygen compound of formula 22 and/or the tautomer thereof to an amine protecting group-removing reaction as shown to give the imine compound of formula I:

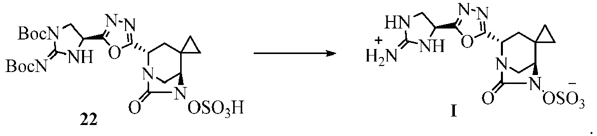

17. The preparation method according to claim 16, wherein the preparation method meets one or more of the following conditions:
(1) in scheme 1, the reaction operations and conditions in the preparation method are the same as the corresponding reaction operations and conditions in the preparation method according to any one of claims 1 to 2;
(2) in scheme 2, the reaction operations and conditions in the preparation method are the same as the corresponding reaction operations and conditions in the preparation method according to any one of claims 1 to 2; and
(3) in scheme 3, the reaction operations and conditions in the preparation method are the same as the corresponding reaction operations and conditions in the preparation method according to any one of claims 1 to 2.
